(19) Europäisches Patentamt
European Patent Office
Office européen des brevets

(11) **EP 3 603 663 A1**

(12) **EUROPEAN PATENT APPLICATION**
published in accordance with Art. 153(4) EPC

(43) Date of publication:
**05.02.2020 Bulletin 2020/06**

(21) Application number: **18776532.6**

(22) Date of filing: **29.03.2018**

(51) Int Cl.:
***A61K 39/00*** *(2006.01)* ***C07K 16/18*** *(2006.01)*
***C07K 1/04*** *(2006.01)* ***C07K 1/18*** *(2006.01)*

(86) International application number:
**PCT/CN2018/081080**

(87) International publication number:
**WO 2018/177369 (04.10.2018 Gazette 2018/40)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **30.03.2017 CN 201710202043**
**11.04.2017 CN 201710233373**
**16.05.2017 CN 201710342257**

(71) Applicants:
- **Jiangsu Hengrui Medicine Co., Ltd.**
  **Jiangsu 222047 (CN)**
- **Shanghai Hengrui Pharmaceutical Co., Ltd**
  **Shanghai 200245 (CN)**

(72) Inventors:
- **LIU, Yupeng**
  **Shanghai 200245 (CN)**
- **ZHANG, Xiaofei**
  **Shanghai 200245 (CN)**
- **LIANG, Zhi**
  **Shanghai 200245 (CN)**
- **SHI, Ruijun**
  **Shanghai 200245 (CN)**
- **ZHONG, Jin**
  **Shanghai 200245 (CN)**
- **LIU, Xun**
  **Shanghai 200245 (CN)**
- **TAO, Weikang**
  **Shanghai 200245 (CN)**
- **ZHANG, Lianshan**
  **Shanghai 200245 (CN)**
- **SUN, Piaoyang**
  **Jiangsu 222047 (CN)**

(74) Representative: **dompatent von Kreisler Selting Werner -**
**Partnerschaft von Patent- und Rechtsanwälten mbB**
**Deichmannhaus am Dom**
**Bahnhofsvorplatz 1**
**50667 Köln (DE)**

(54) **METHOD FOR PREPARING ANTIBODY-DRUG CONJUGATE**

(57) A method for preparing an antibody-drug conjugate (ADC). In particular, the method mainly utilizes a combination of antibody biomolecules and an ion exchange carrier through electrostatic interaction to realize solid phase preparation of an ADC drug. Elution conditions are optimized, to control a drug-to-antibody coupling ratio (DAR) and separate a polymer-coupled drug, reduce the amount of a drug used in a coupling reaction, and enhance the targeted therapeutic effect of an ADC drug. The preparation method features fewer steps, simple operation, and programmable control, facilitating industrial scale-up production, and also realizing zero retention of reducing agents and organic solvents in the preparation process, significantly improving drug safety and reducing production costs.

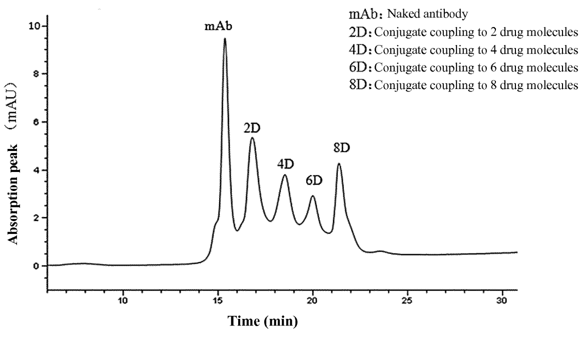


Figure 10

EP 3 603 663 A1

## Description

The present invention claims the priority from Chinese patent application NO: CN201710202043.1 filed on March 30th, 2017, Chinese patent application NO: CN201710233373.7 filed on April 11th, 2017 and Chinese patent application NO: CN201710342257.9 filed on May 16th, 2017. The entire content of which are hereby incorporated by reference.

## Field of the invention

The present invention relates to a method for preparating an antibody-drug conjugate, specifically relates a method for preparating an antibody-drug conjugate (ADC) using ion exchange column as carrier.

## Background

Conventional coupling methods for preparating an ADC drug include: lysine coupling, light-and-heavy interchain reductive disulfide bridges coupling and site-directed coupling (Beck A, Reichert JM. Antibody-drug conjugates: Present and future; MAbs, 2014, 6: 15-17; McCombs J R, Owen S C. Antibody-drug conjugates: design and selection of linker, payload and conjugation chemistry. The AAPS journal, 2015, 17: 339-351). The lysine coupling platform technology utilizes a bifunctional group crosslinking reagent to randomly modify the lysine residue of the antibody and then react with mercapto group of toxic small molecule such as the maytansin derivative DM1, DM4 or the like to achieve coupling. T-DM1 (Kadcyla), which is already on the market, adopts this technology. Light-and-heavy interchain reductive disulfide bridges coupling is achieved by reducing the disulfide bridges between the light chain and heavy chain of the antibody to produce cysteine residues, and then reacting with toxin containing polypeptide-crosslinker such as aplysiatoxin derivative Methylauristatin E (MMAE) or other analogues for coupling. ADC drug Adcetris, which is already on the market, adopts this technology. The site-directed coupling mainly modifies the amino acid sequence by introducing a new amino acid such as cysteine, so that the toxic small molecule is directionally bound to the antibody. The technique is currently at the early stage of development (Panowski S, et al. Site-directed antibody-drug conjugates for cancer therapy. MAbs. Taylor & Francis, 2014, 6: 34-45). At present, ADC drugs on the market are mainly based on lysine coupling and light-and-heavy interchain reductive disulfide bridges random coupling. ADC drugs obtained by these two methods have a large difference in the number of drugs coupled to the monoclonal antibody, resulting in a heterogeneity of drugs, which is a great challenge to the consistency of ADC batch production (Wang L, et al. Structural characterization of the maytansinoid-monoclonal antibody immunoconjugate, huN901-DM1, by mass spectrometry. Protein science, 2005, 14: 2436-2446). The drug toxin-antibody coupling ratio (DAR) represents the average number of toxic drug small molecules coupled to each antibody. Studies have shown that the number of toxic molecules coupled to the antibody affects the polymerization of the ADC, the activity of binding antigen, clearance in the blood circulation, and activity and tolerance of ADC. A DAR value that is too low will reduce its activity, while DAR value that is too high will reduce the half-life and tolerance of the ADC drug in the blood circulation, which will impair the effective binding of the ADC drugs to the antigens. Meanwhile, the efficacy of drug may also be reduced with the increase of DAR value (Hamblett KJ, et al. Effects of drug loading on the antitumor activity of a monoclonal antibody-drug conjugate. Clin Cancer Res, 2004, 10: 7063-7070). For ADC drugs currently available on the market, the ideal DAR value should be controlled between 2 and 4.

Although the development of new ADC drugs has achieved unprecedented success, the technique still needs to be further improved. Among them, the traditional process contains too many coupling steps and complicated operation, which may lead to environmental pollution. It also involves the addition and stirring of organic solvents, which inevitably leads to the collisions between antibody proteins and the production of crosslinking compounds and polymers. Moreover, it is difficult to completely remove various organic solvents, which may cause immunogenicity and other side effects of ADC drugs, thereby constraining its rapid development. In addition, the DAR value is also a key quality control parameter in the preparation of ADC drugs, which also requires research on the coupling and purification process. The DAR value is controlled within the target range to ensure consistency and stability of batch production, and the content of by-product aggregation should be controled and removed as much as possible.

The patent (CN104208719A) gained control of DAR value and polymer by cation exchange chromatography purification, however, the patent only provided a means of purification and separation, which did not change the complexity of the ADC production process, nor could it completely remove various organic reagents.

In term of the modification of process, Evans from UK invented a solid phase preparation of ADC (CN105579066A). He used the affinity filler Protein A resin as a fixative and bound the monoclonal antibody to Protein A through its affinity to the resin, then the monoclonal antibody reacted with crosslinker or toxins, respectively. This research did reduce the production steps, but due to the high cost and poor alkali resistance of Protein A affinity fillers, the spreading and application of this technology in production was severely restricted.

Patent CN101087611A, the entire content of which is hereby incorporated by reference, discloses a method

of coupling an antibody to DM1, DM3, DM4 by a crosslinker comprising maleimide group, mercapto group, and haloacetyl group; wherein the maleimide group is selected from SMCC, LC-SMCC, KMUA, GMBS, EMCS, MBS, AMAS, SMPH, SMPB and PMPI. The haloacetyl group is selected from SIAB, SIA, SBA and SBAP.

Patent CN106029083A, the entire content of which is hereby incorporated by reference, discloses a hydrolyzed succinimide ring (or succinic acid) that directly couples MMAE, MMAF to an antibody via a thioether bond.

Patent CN106467575A, the entire content of which is hereby incorporated by reference, discloses the coupling of antibodies to toxins such as MMAE, MMAF, PBD, SN-38 and Dox by site-directed coupling.

Applicant's prior applications WO2016127790A1 and WO2015/113476 involve new toxin molecules and conjugates, the entire content of which are hereby incorporated by reference. Applicant's prior application CN106188293A discloses a c-Met antibody conjugate and a preparation method thereof. Applicant's prior application (application number CN201610526367.6) provides an EGFR antibody-drug conjugates and a preparation method thereof.

Therefore, a new process, which could not only cut down production steps and reduce costs, but also facilitate the removal of organic reagents and improve the controllability of DAR value, will have important practical significance for ADC drug synthesis.

## Detailed Description of the Invention

The present invention provides a method for preparing an antigen-binding protein-drug conjugate (ADC), which realizes solid phase preparation of ADC drug by utilizing a combination of antibody biomolecule and ion exchange carrier through electrostatic interaction. Elution conditions are optimized, to control drug-to-antibody coupling ratio (DAR) and separate polymer-coupled drug, reduce the amount of drug used in coupling reaction, and enhance the targeted therapeutic effect of an ADC drug. The preparation method features fewer steps, simple operation and programmable control, facilitating industrial scale-up production, and allows realizing zero retention of reducing agents and organic solvents in the preparation process, significantly improving drug safety and reducing production cost. The preparation method comprises: immobilizing an antigen-binding protein on an ion exchange carrier, connecting drug to the immobilized antigen-binding protein by coupling, the coupling product is eluted from the ion exchange carrier, and the eluate is collected; The antigen-binding protein-drug conjugate can be specifically an antibody-drug conjugate.

To achieve above objectives, the technical solution of the present invention is: a method for preparing an antigen-binding protein-drug conjugate (ADC), which comprises the following steps:

1) immobilizing antigen-binding protein on ion exchange carrier to form an immobilized antigen-binding protein;
2) contacting the immobilized antigen-binding protein with drug to form an antigen-binding protein-drug conjugate;
3) eluting the antigen-binding protein-drug conjugate from the ion exchange carrier.

Preferably, the method comprises the following steps:

1) immobilizing antigen-binding protein on ion exchange carrier to form an immobilized antigen-binding protein;
2) contacting the immobilized antigen-binding protein with drug for a coupling reaction to form an antigen-binding protein-drug conjugate; and the coupling reaction is performed to couple the immobilized antigen-binding protein to the drug;
3) eluting the antigen-binding protein-drug conjugate from the ion exchange carrier.

In a preferred embodiment of the present invention, wherein the antigen-binding protein is selected from the group consisting of a humanized antibody, a murine antibody, a human antibody, a chimeric antibody, a single chain antibody, and a bispecific antibody. The antigen-binding protein may also be an antigen-binding fragment selected from, but not limited to, Fab, F(ab')2 and scFv fragments.

In a preferred embodiment of the present invention, wherein the antigen-binding protein is preferably selected from an antibody, more preferably a monoclonal antibody.

In a preferred embodiment of the present invention, the ion exchange carrier is selected from the group consisting of, but not limited to, an ion exchange resin, an ion exchange membrane, an ion exchange fiber, preferably an ion exchange resin. The ionic exchange carrier is selected from a cation exchange carrier or an anion exchange carrier, preferably a cationic exchange carrier, more preferably a strong cation exchange carrier, and the ionic exchange carrier exchanger or reaction ligand is selected from highly acidic reaction ligands, preferably a sulfonic acid group ($-SO_3H$). The matrix used in the ion exchange carrier comprises a hydrophobic matrix, which may be selected from, but not limited to, hydrophobic high molecular polymers such as polystyrene and polymethacryl, preferably polymethacrylate; and hydrophilic matrix, which may be selected from, but not limited to, a hydrophilic polymer such as agarose or dextran, preferably agarose; the matrix used in the ion exchange carrier may also include a polymer of a neutral matrix. In some embodiments, 'ion exchange carrier' and 'carrier' may be used interchangeably and have the same meaning.

In a preferred embodiment of the present invention, wherein the coupling reaction in step 2) is such that the antigen-binding protein and the drug are linked by an interaction between a nucleophilic group and an electrophilic group;

Wherein the nucleophilic group is optionally derived from the antigen-binding protein or the drug, preferably from the antigen-binding protein;

Wherein the electrophilic group is optionally derived from the antigen-binding protein or the drug, preferably from the drug.

In a preferred embodiment of the present invention, wherein the nucleophilic group is selected from the group consisting of a mercapto, a hydroxyl group, an amino group, a hydrazide, a oxime, a hydrazine, a thiosemicarbazone, a hydrazine carboxylate and an aryl hydrazide group, provided that:

When the nucleophilic group is derived from the drug, the nucleophilic group is preferably a mercapto;

When the nucleophilic group is derived from the antigen-binding protein, the nucleophilic group is preferably an amino group (e.g. Embodiment 2 of the present invention), a mercapto (e.g. Embodiment 9 of the present invention), a hydroxyl group; and the amino group is more preferably an N-terminal amino group, a side chain amino group, an amino group of the saccharide in the glycosylation antigen-binding protein, the hydroxyl group is more preferably a hydroxyl group of the saccharide in the glycosylation antigen-binding protein, and the mercapto group is more preferably a thiol side chain, and most preferably a thiol side chain of a cysteine.

In a preferred embodiment of the present invention, wherein the mercapto is derived from a linker produced by cleavage reduction of an antibody, and the amino group is derived from its own linker of an antibody which has not undergone a cleavage reduction.

In a preferred embodiment of the invention, wherein the electrophilic group is selected from the group consisting of an active ester, a hydrocarbyl halide, a benzyl halide, an aldehyde, a ketone, a carboxyl group and a maleimide group, provided that:

When an electrophilic group is derived from the antigen-binding protein, the electrophilic group is preferably derived from an aldehyde, a ketone, a carboxyl group, and a maleimide group, more preferably a maleimide group;

When the electrophilic group is derived from the drug, the electrophilic group is preferably an active ester, a hydrocarbyl halide, a maleimide group, more preferably a maleimide group; the active ester is preferably an NHS ester, an HOBt ester, a haloformate, an acid halide, the hydrocarbyl halide is preferably a haloacetamide.

In a preferred embodiment of the invention, wherein the electrophilic group is derived from the drug itself or from a modification of the drug.

In a preferred embodiment of the present invention, wherein the coupling reaction is selected from the group consisting of lysine coupling, light-and-heavy interchain reductive disulfide bridge coupling or site-directed coupling, preferably lysine coupling, light-and-heavy interchain reductive disulfide bridge coupling. The light-and-heavy interchain reductive disulfide bridge coupling described in the present invention includes reductive disulfide bridge coupling between a light chain and a heavy chain, and also includes reductive disulfide bridge coupling between heavy chains.

In a preferred embodiment of the present invention, wherein the conductivity of the antigen-binding protein of step 1) is adjusted to less than 5 mS/cm prior to contacting with the ion exchange carrier.

In a preferred embodiment of the present invention, wherein the antigen-binding protein of step 1) is immobilized on an ion exchange carrier in a buffer having a pH of 5.5 to 7.0, preferably 6.3, wherein the buffer is selected from the group consisting of phosphate buffer, acetate buffer, citrate buffer, succinate buffer, preferably phosphate buffer.

In a preferred embodiment of the present invention, in the coupling reaction described in the step 2), the molar ratio of the drug to the antigen-binding protein is controlled to be less than 6:1 by slowly flowing the drug through the ion exchange carrier with flow rate of 0.2-2 ml/min. The reaction temperature is room temperature, which is 10-37°C; and the value of temperature is an integer or a decimal, and preferably 20-25°C in non-limiting embodiments; conventional temperature for industrial production is 25°C. Higher temperature will increase the formation of polymers.

In a preferred embodiment of the present invention, wherein the eluting of step 3) comprises stepwise elution using buffers with different salt concentrations, and the buffer has a pH of 5.0 to 6.5, preferably 5.5. The buffer is selected from the group consisting of phosphate buffer, acetate buffer, citrate buffer, succinate buffer, preferably citrate buffer.

In a preferred embodiment of the present invention, the stepwise elution comprises a first step of elution and a second step of elution, the salt concentration of the first step elution is 100-140 mM, preferably 110 mM, the salt concentration of the second step elution is 150-200 mM, preferably 180 mM.

The invention also relates to an optimized method for preparing an antigen-binding protein-drug conjugate (ADC), which involves binding an antigen-binding protein to a crosslinker to produce a nucleophilic group. The illustrative embodiments in the present invention are embodiment 2, 3, 4, 5 and 6; comprising:

1) immobilizing an antigen-binding protein on an ion exchange carrier to form a immobilized antigen-binding protein;
2) contacting the immobilized antigen-binding protein with a drug for coupling reaction to form an antigen-binding protein-drug conjugate;
3) eluting the antigen-binding protein-drug conjugate from the ion exchange carrier;

The step 1) described therein includes:

a) binding the antigen-binding protein to the crosslinker to obtain a modified antigen-binding protein;
b) immobilizing the modified antigen-binding protein on an ion exchange carrier to form a immobilized antigen-binding protein.

In a preferred embodiment of the present invention, wherein the temperature for binding the antigen-binding protein to the crosslinker is 20 to 40°C.
In a preferred embodiment of the present invention, wherein the binding of the antigen-binding protein to the crosslinker is carried out in a buffer having a pH of 4.0 to 5.5, preferably 4.3; the buffer is preferably an acetate buffer, more preferably an acetate buffer containing acetonitrile.
In a preferred embodiment of the present invention, wherein the step 1) further comprises step c:
c. adding a deprotecting agent, and the deprotecting agent is preferably $NH_2OH{\cdot}HCL$.
The present invention also relates to another optimized method for preparing an antigen-binding protein-drug conjugate (ADC), which involves reducing an antigen-binding protein to produce a nucleophilic group, and a illustrative embodiment of which in the present invention is embodiment 9. The method comprises: 1) immobilizing an antigen-binding protein on an ion exchange carrier to form a immobilized antigen-binding protein;

2) contacting the immobilized antigen-binding protein with a drug for coupling reaction to form an antigen-binding protein-drug conjugate;
3) eluting the antigen-binding protein-drug conjugate from the ion exchange carrier;

The step 1) described therein includes:

A. immobilizing the antigen-binding protein on an ion exchange carrier to form a immobilized antigen-binding protein;
B. adding a reducing agent to reduce the disulfide bridge of the immobilized antigen-binding protein to produce a mercapto (a nucleophilic group thiol for coupling with a drug);

Wherein the reducing agent is preferably selected from the group consisting of TCEP, DTT, mercaptoethylamine, Ac-Cys, more preferably TCEP. The reducing agent is used in an amount of 4-8 times the molar concentration of the antibody, preferably 6 times the molar concentration of the antibody.
In a preferred embodiment of the present invention, the temperature of reduction reaction in step B is 25 to 45°C, preferably 40°C.
In the invention, the drug includes a toxin (for example, an enzymatically active toxin or a fragment thereof derived from bacteria, fungi, plant or animal), a chemotherapeutic agent, a growth inhibitor, a tubulin inhibitor, an antibiotic, a radioisotope, a nucleolytic enzyme and other cytotoxic agents; the drug needs to be capable of removing a tumor by inhibiting microtubules or cleaving DNA of a tumor cell; the toxin includes, but is not limited to, a small molecule drug such as camptothecin derivatives, calicheamicin, maytansinoids, dolastatin, auristatin, trichothecene, and cytotoxicly activated fragments of these drugs; the drug is selected from the group consisting of a derivative of maytansinoids, preferably DM1, DM3, DM4; may also be selected from auristatin derivatives, preferably MMAE, MMAF; or may be selected from camptothecin alkaloids, preferably CPT, 10-hydroxy-CPT, CPT -11 (irinotecan), SN-38 and topotecan, more preferably SN-38.
In the method of the present invention, the antigen-binding protein binds to one or more polypeptides selected from the group consisting of: HER2, HER3, CD33, VEGF, VEGFR, VEGFR-2, CD152, CD40, TNF, IL-1, IL-5, IL-17, IL-6R, IL-1, IL-2R, BLYS, OX40L, CTLA4, PCSK9, EGFR, c-Met, CD2, CD3, CD11a, CD19, CD30, CD38, CD20, CD52, CD60, CD80, CD86, TNF-$\alpha$, IL-12, IL-17, IL-23, IL-6, IL-1$\beta$, RSVF, IgE, RANK, BLyS, $\alpha4\beta7$, PD-1, CCR4, SLAMF7, GD2, CD21, CD79b, IL20R$\alpha$, brevican, CD22, CD79a, CD72, IGF-1R and RANKL
In the method of the present invention, the antigen-binding protein may further be selected from the group consisting of: Humira (adalimumab), Avastin (bevacizumab), Erbitux (cetuximab), Herceptin (Trastuzumab), Perjeta (Pertuzumab), Vectibix (Panibizumab), Theraloc (Netuzumab), Yervoy (Ipilimumab), Opdivo (Navolumab), Lucentis (Ranibizumab), Enbrel (Enacept), Myoscint (Imciromab pentetate), ProstaScint (Capromab pendetide), Remicade (Infliximab), ReoPro (Abciximab), Rituxan (rituximab), Simulect (Basiliximab), Synagis (Palivizumab), Verluma (Nofetumomab), Xolair (Omalizumab), Zenapax (Daclizumab), Cimzia (certolizumab), Zevalin (Ibritumomab), Orthoclone (Morommonab), Panorex (Edrecolomab), Mylotarg (Gemtuzumab), Soliris (Eculizumab), CNTO1275 (ustekinumab), Amevive (Alefacept), Raptiva (Efalizumab), Tysabri (Natalizumab), Acternra (Tocilizumab), Orencia (Abatacept), Arcalyst (Rilonacep), Stelara (Ustekinumab), Removab (Catumaxomab), Simponi (Golimumab), Ilaris (Canakinumab), Arzerra (Ofatumumab), Prolia (Denosumab), Benlysta (B elimumab), Nulojix (Belatacept), Eylea (Aflibercept), Campath (Alemtuzumab), CEA-Scan arcitumomab (fab fragment), Potelige (mogamulizumab), Abthrax (Raxibacumab), Gazyva (O

binutuzumab), Lang Mu (Conbercept), Cyramza (Ramucirumab), Sylvant (Siltuximab), Entyvio (Vedolizumab), Keytruda (Pembrolizumab), Blincyto (Blinatumonab), Cosentyx (Secukinumab), Unituxin (Dinutuximab), Darzalex (Daratumumab), Praluent (Alirocumab), Repatha (Evolocumab), Portrazza (Necitumumab), Empliciti (Elotuzumab), Nucala (M epolizumab), Praxbind (Idarucizumab), Bexxar (Tositumomab and 1131 Tositumomab).

In some embodiments, the antigen-binding protein is selected from an anti-EGFR antibody or antigen-binding fragment thereof, and the anti-EGFR antibody or antigen-binding fragment thereof, preferably comprises LCDR1, LCDR2 and LCDR3 regions having the sequence of SEQ ID NO: 5, SEQ ID NO: 6, SEQ ID NO:7 and its variants, and HCDR1, HCDR2, HCDR3 regions having the sequence of SEQ ID NO:8, SEQ ID NO:9, SEQ ID NO:10 and their variants, more preferably the light chain of SEQ ID NO: 1 and the heavy chain of SEQ ID NO: 2.

| | |
|---|---|
| mAb001-LCDR1: RSSQNIVHSNGNTYLD | SEQ ID NO:5 |
| mAb001-LCDR2: KVSNRFS | SEQ ID NO:6 |
| mAb001-LCDR3: FQYSHVPWT | SEQ ID NO:7 |
| mAb001-HCDR1: NYYIY | SEQ ID NO:8 |
| mAb001-HCDR2: GINPTSGGSNFNEKFKT | SEQ ID NO:9 |
| mAb001-HCDR3: QGLWFDSDGRGFDF | SEQ ID NO:10 |

The amino acid sequence of mAb001 light chain is set forth in SEQ ID NO:1

DIQMTQSPSSLSASVGDRVTITCRSSQNIVHSNGNTYLDWYQQTPGKAPKLLIYK
VSNRFSGVPSRFSGSGSGTDFTFTISSLQPEDIATYYCFQYSHVPWTFGQGTKLQIT
RTVAAPSVFIFPPSDEQLKSGTASVVCLLNNFYPREAKVQWKVDNALQSGNSQES
VTEQDSKDSTYSLSSTLTLSKADYEKHKVYACEVTHQGLSSPVTKSFNRGEC

The amino acid sequence of mAb001 heavy chain is set forth in SEQ ID NO:2

QVQLQQSGAEVKKPGSSVKVSCKASGYTFTNYYIYWVRQAPGQGLEWIGGINPT
SGGSNFNEKFKTRVTITADESSTTAYMELSSLRSEDTAFYFCTRQGLWFDSDGRGF
DFWGQGTTVTVSSASTKGPSVFPLAPSSKSTSGGTAALGCLVKDYFPEPVTVSWN
SGALTSGVHTFPAVLQSSGLYSLSSVVTVPSSSLGTQTYICNVNHKPSNTKVDKKV
EPKSCDKTHTCPPCPAPELLGGPSVFLFPPKPKDTLYITREPEVTCVVVDVSHEDP
EVKFNWYVDGVEVHNAKTKPREEQYNSTYRVVSVLTVLHQDWLNGKEYKCKV
SNKALPAPIEKTISKAKGQPREPQVYTLPPSRDELTKNQVSLTCLVKGFYPSDIAVE
WESNGQPENNYKTTPPVLDSDGSFFLYSKLTVDKSRWQQGNVFSCSVMHEALH
NHYTQKSLSLSPGK

In other embodiments, the antigen-binding protein may also be selected from an anti-c-Met antibody or antigen-binding fragment thereof, preferably comprising LCDR1, LCDR2, LCDR3 regions having the sequence of SEQ ID NO: 11 or SEQ ID NO: 17, SEQ ID NO: 12, SEQ ID NO: 13 and variants thereof, preferably a LCDR1 of SEQ ID NO: 17, and HCDR1, HCDR2, HCDR3 regions of SEQ ID NO: 14, SEQ ID NO: 15, SEQ ID NO: 16 and of their variants, respectively; more preferably a light chain of SEQ ID NO: 3 and a heavy chain of SEQ ID NO: 4.

| | |
|---|---|
| mAb002-LCDR1: RANKSVSTSTYNYLH | SEQ ID NO: 11 |
| mAb002-LCDR2: LASNLAS | SEQ ID NO: 12 |

(continued)

| | |
|---|---|
| mAb002-LCDR3: QHSRDLPPT | SEQ ID NO: 13 |
| mAb002-HCDR1: NYGVH | SEQ ID NO: 14 |
| mAb002-HCDR2: VIWSGGSTNYAAAFVS | SEQ ID NO: 15 |
| mAb002-HCDR3: NHDNPYNYAMDY | SEQ ID NO: 16 |
| optimized mAb002-LCDR1: RADKSVSTSTYNYLH | SEQ ID NO: 17 |

The amino acid sequence of mAb002 light chain is set forth in SEQ ID NO: 3

DIVLTQSPDSLAVSLGERATINCRADKSVSTSTYNYLHWYQQKPGQPPKLLIYLAS
NLASGVPDRFSGSGSGTDFTLTISSLQAEDVAVYYCQHSRDLPPTFGQGTKLEIKR
TVAAPSVFIFPPSDEQLKSGTASVVCLLNNFYPREAKVQWKVDNALQSGNSQESV
TEQDSKDSTYSLSSTLTLSKADYEKHKVYACEVTHQGLSSPVTKSFNRGEC

The amino acid sequence of mAb002 light chain is set forth in SEQ ID NO: 4

QVQLVESGGGVVQPGRSLRLSCAASGFSLSNYGVHWVRQAPGKGLEWLAVIWS
GGSTNYAAAFVSRLTISKDNSKNTVYLQMNSLRAEDTAVYYCARNHDNPYNYA
MDYWGQGTTVTVSSASTKGPSVFPLAPCSRSTSESTAALGCLVKDYFPEPVTVS
WNSGALTSGVHTFPAVLQSSGLYSLSSVVTVPSSNFGTQTYTCNVDHKPSNTKVD
KTVERKCCVECPPCPAPPVAGPSVFLFPPKPKDTLMISRTPEVTCVVVDVSHEDPE
VQFNWYVDGVEVHNAKTKPREEQFNSTFRVVSVLTVVHQDWLNGKEYKCKVS
NKGLPAPIEKTISKTKGQPREPQVYTLPPSREEMTKNQVSLTCLVKGFYPSDIAVE
WESNGQPENNYKTTPPMLDSDGSFFLYSKLTVDKSRWQQGNVFSCSVMHEALH
NHYTQKSLSLSPGK

In the method of the present invention, the drug is a modified or unmodified drug, preferably a modified one; the antigen-binding protein is a modified or unmodified antigen-binding protein, preferably a modified one. The coupling between the drug and the antigen-binding protein is formed by the interaction of the electrophilic group with the nucleophilic group, or the interaction between the nucleophilic group and the electrophilic group. The chemical agent for modificating the drug and the antigen-binding protein may be any substance capable of forming two kind of groups described above, including a crosslinker, by which an effective linker between the antigen-binding protein and the drug can be formed to generate an ADC drug.

In the method of the present invention, the linker can effectively connect the antigen-binding protein and the drug, and the synthesized ADC drug can self-break in the human body without any toxic and side effects, exerts an effective cytotoxic effect of the drug on tumor cells.

In the treatment using ADC drugs, linker plays an important role, it not only ensures the stability of the drug in the blood flow, but also ensures a quick and efficient release of the drug in the tumor cells. Currently two main types of linkers, cleavable and uncleavable, were used in ADC drugs. When using leavable linker, ADC release toxin drug by acid hydrolysis or specific protease cleavage in cell endosome or lysosome; when using uncleavable linker, ADC that is endocytosed into cell needs to be digested and degraded to release small molecule drug. The cleavable linker includes: a hydrazone linker which is cleaved under acidic conditions, a disulfide bridge linker which hydrolyzes under the action of a reducing substance such as glutathione, a protease-hydrolyzed polypeptide linker (Val-Cit, Phe-Lys) and a β-glucoside linker, etc.; uncleavable linker mainly includes a linker which can form a thioether bond, and the like. Both the cleavable linker and uncleavable linker described above, preferably a uncleavable linker, is suitable for use in the method

of the present invention.

In the method of the present invention, the crosslinker used for forming the linker may be a homobifunctional crosslinker, preferably an amino-amino crosslinker, which has two identical activating reactive groups at both ends, mainly N-Hydroxysuccinimide esters and imidoesters, which can react with free primary amines of the basic amino acids on the surface of the protein, e.g. disuccinimidyl glutarate (DSG) of succinimide, or imidate such as disuccinimidyl 3,3'-Dithiodipropioniate (DSP), dimethyl 3,3'-dithiobispropioninimidate (DTBP) etc.

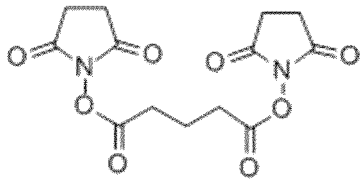

(DSG)

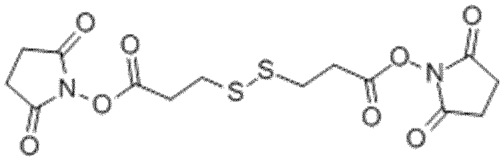

(DSP)

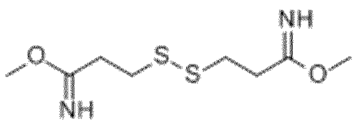

(DTBP)

It may also be a heterobifunctional crosslinker with two different activating reactive groups at both ends, which can react with other groups of different types, mainly including N-hydroxysuccinimide-maleimide and N-hydroxysuccinimide-dimercaptopyridine, such as SMCC, LC-SMCC, KMUA, GMBS, EMCS, MBS, AMAS, SMPH, SMPB and PMPI of N-hydroxysuccinimide-maleimide, N-succinimide 3-(2-pyridinedithio)propionate (SPDP), 4-succinimidyloxycarbonyl-alpha-methyl-alpha (2-pyridyldithio) toluene (SMPT) of N-hydroxysuccinimide-dimercaptopyridine.

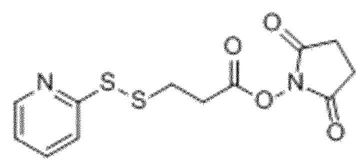

(SPDP)

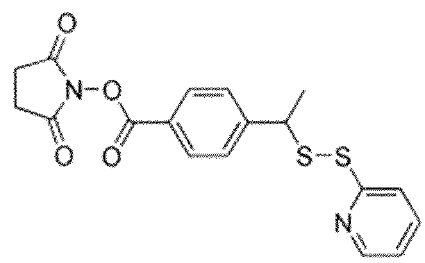

(SMPT)

It may also be another heterobifunctional crosslinker, i.e. a carboxy-amino crosslinker, which is mainly a carbodiimide for coupling with carboxyl group of acidic amino acid at C-terminal and primary ammonia of basic amino acid

at N-terminal. The crosslinker can form an addition product intermediate with a carboxyl group and a carbodiimide, and then react with an amino group to form an amide bond. It mainly includes dicyclohexylcarbodiimide (DCC), 1 -(3-dimethylaminopropyl)-3-ethylcarbodiimide hydrochloride (EDC).

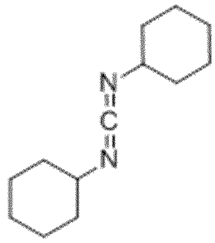

(DCC)

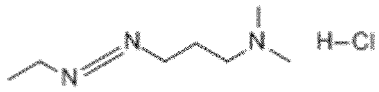

(EDC)

It may also be another heterobifunctional crosslinker with a haloacetyl group, mainly including SIAB, SIA, SBA and SBAP.

In the present invention, certain methods for preparing antibody-drug conjugates by ion exchange carriers can be accomplished by several routes using organic chemical reactions, conditions, and reagents known to those skilled in the art for the combination of antibody and drug, including: (1) performing an reaction of an antibody with a crosslinker via a covalent bond to form an antibody-crosslinker binding product bearing a nucleophilic group or an electrophilic group, followed by reacting with an electrophilic group or a nucleophilic group of the drug; (2) performing an reaction of a drug with a crosslinker via a covalent bond to form a drug-crosslinker binding product bearing a nucleophilic group or an electrophilic group, followed by reacting with an electrophilic group or a nucleophilic group of the antibody; and (3) modifying the antibody to form a nucleophilic group, followed by binding to a drug bearing an electrophilic group. The nucleophilic group or electrophilic group of the above antibodies and drugs may optionally be produced by modification of a chemical agent or a crosslinker without any limitation.

Nucleophilic groups of the antigen-binding protein of the present invention include, but are not limited to, mercapto, hydroxy group, hydrazide, oxime, hydrazine, thiosemicarbazone, hydrazine carboxylate, and aryl hydrazide group, preferably mercapto; the nucleophilic group of the antigen-binding protein is capable of reacting with a crosslinker or an electrophilic group of the drug to form a covalent bond, the electrophilic group of the crosslinker or drug is selected from the group consisting of an active ester, a hydrocarbyl halide, a benzyl halide, an aldehyde, a ketone, a carboxyl group, and a maleimide group, preferably a hydrocarbyl halide or a maleimide group, more preferably a maleimide group; the active ester is preferably an NHS ester, an HOBt ester, halogenated formate, acid halides, and the hydrocarbyl halide is preferably haloacetamide. Or the antigen-binding protein bears an electrophilic group selected from the group consisting of an aldehyde, a ketone, a carboxyl group, and a maleimide group, preferably a maleimide group; wherein the drug bears a nucleophilic group selected from the group consisting of mercapto, hydroxy group, hydrazide, oxime, hydrazine, thiosemicarbazone, hydrazine carboxylate, and aryl hydrazide groups; the antigen-binding protein and the drug described above were combined to form an ADC.

The nucleophilic group of the antigen-binding protein of the present invention may also be selected from the group consisting of an N-terminal amino group, a side chain amino group, a mercapto side chain group, a hydroxyl group or an amino group of saccharide in a glycosylated antigen-binding protein, preferably a mercapto side chain group, more preferably a mercapto of cysteine. Wherein the drug bears an electrophilic group selected from the group consisting of an active ester, a hydrocarbyl halide, a benzyl halide, an aldehyde, a ketone, a carboxyl group, and a maleimide group, preferred hydrocarbyl halide, maleimide group, more preferably maleimide group; the active ester is preferably a NHS ester, a HOBt ester, a haloformate, an acid halide, and the hydrocarbyl halides is preferably a haloacetamide.

The method for coupling the antibody with the drug in the present invention may adopt a lysine coupling or a light-and-heavy interchain reductive disulfide bridge random coupling method, or a site-directed coupling method; different coupling methods are not selective for the antibody itself.

In some embodiments, the drug is selected from the compounds of general formula (Dr):

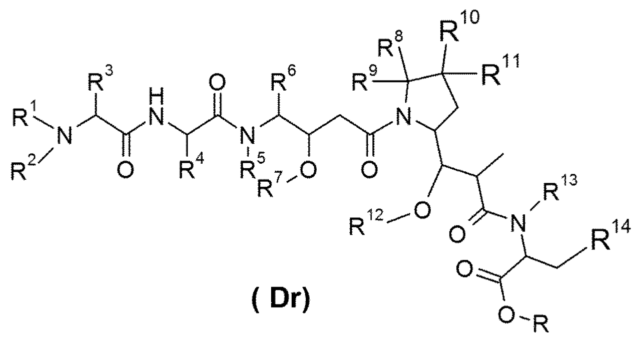

( Dr)

Or a tautomer, a mesomer, a racemate, an enantiomer, a diastereomer, or a mixture thereof, or a pharmaceutically acceptable salt thereof, wherein:

R, $R^1$-$R^7$ are selected from the group consisting of a hydrogen atom, a halogen, a hydroxyl group, a cyano group, an alkyl group, an alkoxy group, and a cycloalkyl group;
At least one of $R^8$-$R^{11}$ is selected from the group consisting of a halogen, a alkenyl, a alkyl and a cycloalkyl, the remainings are hydrogen atoms;
Or any two of $R^8$-$R^{11}$ form cycloalkyl groups, and the remaining two groups are optionally selected from a hydrogen atom, an alkyl group and a cycloalkyl group;
$R^{14}$ is selected from aryl or heteroaryl, and the aryl or heteroaryl is optionally further substituted with the group consisting of a hydrogen atom, a halogen, a hydroxyl group, an alkyl group, an alkoxy group, and a cycloalkyl group;
$R^{12}$-$R^{13}$ are selected from a hydrogen atom, an alkyl group or a halogen;

In some embodiments, the (Dr) compound is modified prior to binding to the antigen-binding protein, preferably having an electrophilic group after modification, followed by reacting with an antigen-binding protein bearing a nucleophilic group to form an ADC; the electrophilic group is preferably a maleimide group or a haloacetyl group, more preferably a maleimide group, and the nucleophilic group is preferably a mercapto group.
In some embodiments, the (Dr) compound is modified prior to binding to the antigen-binding protein, preferably bearing a nucleophilic group after modification, followed by reacting with an antigen-binding protein bearing an electrophilic group to form an ADC; the nucleophilic group is a mercapto, and the electrophilic group of the antigen-binding protein is preferably a maleimide group or a halogenated acetyl group.
In some preferred embodiments, the drug is selected from the compounds of formula (I):

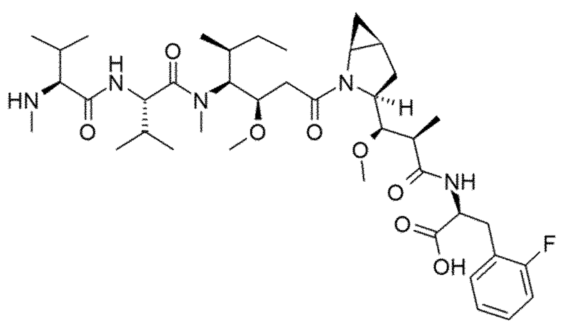

(I)

In some preferred embodiments, the compound of formula (I) is modified prior to binding to the antigen-binding protein, preferablyhaving an electrophilic group after modification, followed by reacting with an antigen-binding protein bearing a nucleophilic group to form an ADC; the electrophilic group is preferably a maleimide group or a haloacetyl group, more preferably a maleimide group, preferably a mercapto group.
In some embodiments, the compound of formula (I) is modified prior to binding to the antigen-binding protein, preferablyhaving a nucleophilic group after modification, followed by reacting with an antigen-binding protein bearing an electrophilic group to form an ADC; the nucleophilic group is a mercapto group, and the electrophilic group of the antigen-binding protein is preferably a maleimide group or a halogenated acetyl group.
In other embodiments, the modified drug is selected from the compounds of formula (L1-Dr):

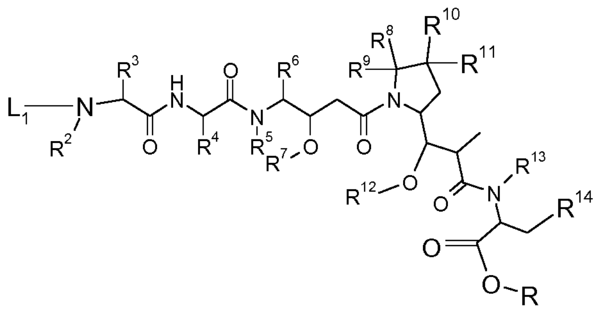

(L1-Dr)

Among them
Wherein the L1 structure is as follows:

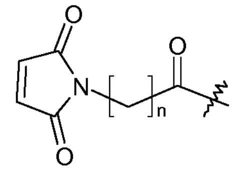 ; preferably MC: 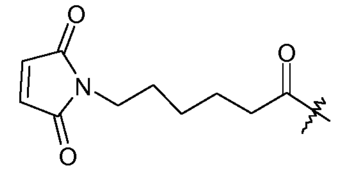 ;

Specifically:

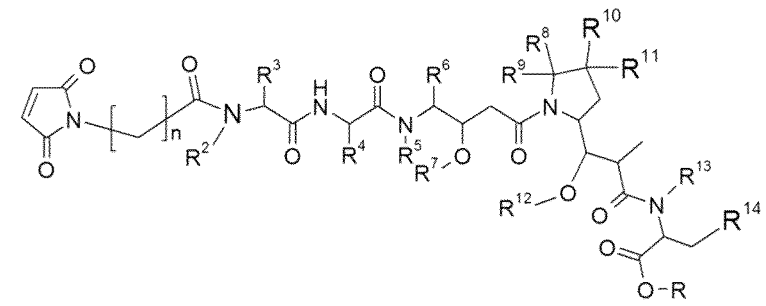

(L1-Dr)

Wherein,

n is 2-6, preferably 2-5;
R, $R^2$-$R^7$ are selected from the group consisting of a hydrogen atom, a halogen, a hydroxyl group, a cyano group, an alkyl group, an alkoxy group, and a cycloalkyl group;
At least one of $R^8$-$R^{11}$ is selected from the group consisting of a halogen, a alkenyl, a alkyl and a cycloalkyl, the remainings are hydrogen atoms;
Or any two of $R^8$-$R^{11}$ form a cycloalkyl group, and the remaining two groups are optionally selected from a hydrogen atom, an alkyl group and a cycloalkyl group;
$R^{14}$ is selected from aryl or heteroaryl, and the aryl or heteroaryl is optionally further substituted with the group consisting of a hydrogen atom, a halogen, a hydroxyl group, an alkyl group, an alkoxy group, and a cycloalkyl group.

In other preferred embodiments, the drug is selected from the compounds of formula (II):

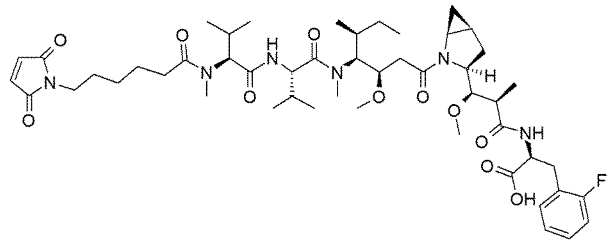

(II)

In the method of the present invention, the solid phase preparation of ADC drug is preferably realized by immobilizing the antigen-binding protein by an ion exchange carrier by using a lysine coupling method, wherein the antibody is modified by using a crosslinker and then combined with the drug to form an ADC. The modification comprises optionally a modification before or after the antigen-binding protein is immobilized on the ion exchange carrier, preferably a modification before the antigen-binding protein is immobilized on the ion exchange carrier.

In a preferred embodiment of the present invention, wherein the antigen-binding protein in step 1) is optionally a modified antigen-binding protein or an unmodified antigen-binding protein, preferably a modified antigen-binding protein; the modified antigen-binding protein optionally binds the antigen-binding protein to a chemical reagent or a crosslinker, preferably a modified antigen-binding protein bound to a crosslinker;

Wherein the drug in step 2) is optionally modified or unmodified, preferably modified.

In some embodiments, the antigen-binding protein is modified with a crosslinker prior to binding of the antigen-binding protein to the ion exchange carrier, followed by immobilizating the modified antigen-binding protein to an ion exchange carrier, followed by binding to the drug to form an ADC.

In one embodiment, the crosslinker has a compound of the formula (L2):

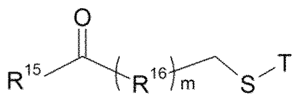

($L_2$)

T is selected from H, tert-butyl, acetyl, n-propionyl, isopropionyl, triphenylmethyl, methoxymethyl, or 2-(trimethylsilyl)ethoxymethyl, preferably H or acetyl ;

$R^{15}$ is selected from the group consisting of a hydrogen atom, a halogen, a hydroxyl group, a cyano group, an alkyl group, an alkoxy group and a cycloalkyl group;

$R^{16}$ is selected from the group consisting of alkyl, cycloalkyl and heterocyclic;

m is 0-5, preferably 1-3;

Wherein the drug has an electrophilic group selected from the group consisting of an active ester, a hydrocarbyl halide, a benzyl halide, an aldehyde, a ketone, a carboxyl group, and a maleimide group, preferred hydrocarbyl halide or maleimide group, more preferably maleimide group; the active ester is preferably NHS ester, HOBt ester, haloformate, acid halide, and the hydrocarbyl halides is preferably haloacetamide.

In a preferred embodiment, the modified drug is selected from the compounds of the formula (L1-Dr).

In a preferred embodiment, the drug is selected from the compound of formula (II).

In another embodiment, the crosslinker has a maleimide group or a moiety of a haloacetyl group; wherein the crosslinker bearing a maleimide group is preferably selected from SMCC, LC-SMCC , KMUA, GMBS, EMCS, MBS, AMAS, SMPH, SMPB, and PMPI, more preferably SMCC; wherein the crosslinker bearing a moiety of a haloacetyl group is preferably selected from SIAB, SIA, SBA and SBAP, more preferably SIAB; wherein the drug bearing a nucleophilic group which preferably selected from mercapto group, hydroxy group, hydrazide, oxime, hydrazine, thiosemicarbazone, hydrazine carboxylate, and aryl hydrazide group, more preferably mercapto group.

In the method of the present invention, a mercapto group can also be formed by reducing an interchain disulfide bridge, i.e. a cysteine bridge, of an antibody, and then reacting with a drug bearing an electrophilic group to form an ADC; the drug may have an own electrophilic group or an electrophilic group modified by a chemical agent, or may has an electrophilic group that is modified by a crosslinker.

In some embodiments, a reducing agent is added to reduce an antigen-binding protein immobilized on an ion exchange carrier; wherein the reducing agent is preferably, but not limited to, tris(2-carboxyethyl)phosphine (TCEP),

dithiothreitol (DTT), mercaptoethylamine, acetylcysteine (Ac-Cys), more preferably tris(2-carboxyethyl)phosphine (TCEP); wherein the drug has an electrophilic group preferably selected from active esters, hydrocarbyl halides, benzyl halides, aldehydes, ketones, carboxyl groups and maleimide groups, preferably hydrocarbyl halides, or maleimide groups, more preferably maleimide groups. The active ester is preferably an NHS ester, an HOBt ester, a haloformate, an acid halide, and the hydrocarbyl halide is preferably a haloacetamide; the drug itself bears an own electrophilic group or an electrophilic group that is modified by a crosslinker, and the modification comprising modification with a chemical reagent or a crosslinker.

In one embodiment, the reducing agent is selected from tri(2-carbonylethyl)phosphine (TCEP), the drug bears an electrophilic group that is modified by a crosslinker, wherein the crosslinker comprises a maleimide group, and the crosslinker comprising a maleimide group is preferably selected from SMCC, LC-SMCC , KMUA, GMBS, EMCS, MBS, AMAS, SMPH, SMPB, and PMPI, more preferably SMCC.

In a preferred embodiment, the reducing agent is selected from tris(2-carboxyethyl)phosphine (TCEP), and the crosslinker has a moiety of haloacetyl group; wherein the crosslinker having a moiety of a haloacetyl group is preferably selected from SIAB, SIA, SBA and SBAP, more preferably SIAB.

In another embodiment, the reducing agent is selected from tris(2-carboxyethyl)phosphine (TCEP), and the drug has an electrophilic group modified by a chemical agent, which is selected from the compounds of formula (Dr).

In another preferred embodiment, the drug is selected from compounds of formula (I).

In another embodiment, the reducing agent is selected from tris(2-carboxyethyl)phosphine (TCEP), and the drug having an own electrophilic group is selected from the compounds of formula (L1-Dr).

In another preferred embodiment, the drug is selected from compounds of formula (II).

The groups in which the antigen-binding protein interacts with the drug in the present invention is mainly a mercapto group and a maleimide (or a halogenated acetyl group), which form a structure of a thioether bond belonging to a uncleavable linker. To form the target linker, a crosslinker can be used to modify the antigen-binding protein or drug, follow by reacting with a reactive group to form an antibody-drug conjugate. Any crosslinker capable of stably linking an antigen-binding protein to a drug is suitable for use in the present invention. The position of the antigen-binding protein to which the drug is attached is interchangeable, and the formation of a thioether bond can also be obtained by reacting a halogenated acetyl group with a free mercapto group.

In the method of the present invention, the method for preparing an ADC using an ion exchange carrier comprises following steps: modifying the antigen-binding protein (for example, an antibody) by a crosslinker, and immobilizing the antigen-binding protein on an ion exchange carrier to form a immobilizedd antigen-binding protein; contacting the immobilized antigen-binding protein with a drug to form an antigen-binding protein-drug conjugate; eluting the antigen-binding protein-drug conjugate from the ion exchange carrier.

In one embodiment, the crosslinker is selected from the compound of formula (L3), the antigen-binding protein comprises LCDR1, LCDR2, LCDR3 region of SEQ ID NO: 5, SEQ ID NO: 6, SEQ ID NO: 7 and variants thereof, and HCDR1, HCDR2, HCDR3 regions of SEQ ID NO: 8, SEQ ID NO: 9, SEQ ID NO: 10 and variants thereof, and the drug is selected from the compound of formula (II).

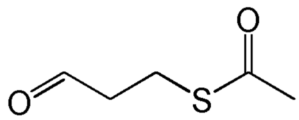

($L_3$)

In a preferred embodiment, the crosslinker is selected from the compound of the formula (L3), the antigen-bingding protein comprises the light chain of SEQ ID NO: 1, and the heavy chain of SEQ ID NO: 2, and the drug is selected from the compound of formula (II).

In another embodiment, the crosslinker is selected from the compound of formula (L3), the antigen-bingding protein comprises LCDR1, LCDR2, LCDR3 regions of SEQ ID NO: 11 or SEQ ID NO: 17, SEQ ID NO: 12, SEQ ID NO: 13 and variants thereof, preferably a LCDR1 of SEQ ID NO: 17, and HCDR1, HCDR2, HCDR3 regions of SEQ ID NO: 14, SEQ ID NO: 15, SEQ ID NO: 16 and variants thereof, and the drug is selected from the compound of formula (II).

In another preferred embodiment, the crosslinker is selected from the compound of the formula (L3), the antigen-bingding protein comprises the light chain of SEQ ID NO: 3, and the heavy chain of SEQ ID NO: 4, and the drug is selected from the compound of formula (II).

The preferred embodiment comprises the steps of: (i) utilizing the mechanism of lysine coupling and the function of reducing agent, the lysine was modified by a crosslinker such that reductive amination of the amino group of the lysine side chain of the antibody with the aldehyde group at the end of the crosslinker was conducted to form an antibody-crosslinker conjugate, and the free crosslinker was removed; (ii) equilibrating the cation exchange column, and rinsing the column with crosslinker, re-equilibrating, followed by loading the antibody-crosslinker conjugate onto an ion exchange column, and rinsing for three times; (iii) deprotecting the terminal of the crosslinker; (iv) equilibrating, and loading toxin onto the ion exchange column, followed by secondary rinsing, secondary eluting, and regeneration .

In an additional method of preparing an ADC of the present invention, an antigen-binding protein is coupled to a drug by a method of reducing an interchain disulfide bridge. After the antigen-binding protein is immobilized on the ion exchange carrier, the step of adding a reducing agent is carried out to reduce the disulfide bridge of the antigen-binding protein to a free mercapto group, which react with a drug bearing a maleimide group or a halogenated acetyl group to form a thioether-linked ADC drug.

In another embodiment, the crosslinker is selected from Tris(2-carboxyethyl)phosphine (TCEP), the antigen-bingding protein comprises LCDR1, LCDR2, LCDR3 regions of SEQ ID NO: 5 or SEQ ID NO: 6, SEQ ID NO: 7 and variants thereof, and HCDR1, HCDR2, HCDR3 regions of SEQ ID NO: 8, SEQ ID NO: 9, SEQ ID NO: 10 and variants thereof, and the drug is selected from the compound of formula (II).

In a preferred embodiment, the crosslinker is selected from Tris(2-carboxyethyl) phosphine (TCEP), the antigen-bingding protein comprises the light chain of SEQ ID NO: 1 and the heavy chain of SEQ ID NO: 2, and the drug is selected from the compound of formula (II).

In another embodiment, the crosslinker is selected from Tris(2-carboxyethyl)phosphine (TCEP), the antigen-bingding protein comprises LCDR1, LCDR2, LCDR3 regions of SEQ ID NO: 11 or SEQ ID NO: 17, SEQ ID NO: 12, SEQ ID NO: 13 and variants thereof, preferably a LCDR1 of SEQ ID NO: 17, an HCDR1, HCDR2, HCDR3 regions of SEQ ID NO: 14, SEQ ID NO: 15, SEQ ID NO: 16 and variants thereof, and the drug is selected from the compound of formula (II).

In another preferred embodiment, the crosslinker is selected from Tris(2-carboxyethyl) phosphine (TCEP), the antigen-bingding protein comprises the light chain of SEQ ID NO: 3 and the heavy chain of SEQ ID NO: 4, and the drug is selected from the compound of formula (II).

A preferred embodiment comprises the steps of: (i) utilizing a interchain disulfide bridge coupling strategy and a cation exchange resin as an immobilized carrier, equilibrating the column, and loading antibody sample, followed by rinsing, reducing interchain disulfide bridge of antibody by adding reducing agent; (ii) loading toxin onto the ion exchange column, followed by secondary rinsing, secondary eluting, and regeneration.

In some embodiments, the reaction temperature for reducing the interchain disulfide bridge of the antibody by reducing agent is 25-45°C, preferably 40°C.

In all embodiments of the invention, the binding of the antigen-binding protein to the carrier is followed by incubating at the temperature of 10°C to 37°C, preferably 25°C, the value of which is an integer or fraction, and the temperature of which in non-limiting embodiments may be 10°C, 11°C, 12°C, 13° C, 14°C, 15°C, 16°C, 17°C, 18°C, 19°C, 20°C, 21°C, 22°C, 23°C, 24°C, 25°C, 26°C, 27°C, 28°C, 29°C, 30°C, 31°C, 32°C, 33°C, 34°C, 35°C, 36°C or 37°C.

Further, the binding of the antigen-binding protein to the carrier is carried out in a buffer having a pH of 5.5-7.0, preferably 6.3, the buffer includes, but is not limited to, phosphate buffer, acetate buffer, citrate buffer, succinate buffer, preferably phosphate buffer.

Further, when loading the antigen-binding protein onto the carrier, an optimized loading flow rate is controlled at 0.1-0.5 ml/min, which is adjusted according to the amount of the ion exchange carrier used in cartain experiment.

Further, it is necessary to adjust the conductivity of the antigen-binding protein solution within a range of 5 mS/cm before loading the antigen-binding protein onto the ion exchange carrier.

Further, the coupling of the antigen-binding protein immobilized on the ion exchange carrier to the drug is carried out on an ion exchange column. In order to reduce the amount of the drug used in the reaction, the drug is loaded onto the ion exchange carrier at a slow flow rate; and the molar ratio of the drug to the antigen-binding protein is 6, 5, 4, 3, 2, 1, preferably 6, and the preferred flow rate is 0.2 ml/min.

Further, the antigen binding protein and drug conjugate needs to be eluted from the ion exchange carrier after binding, and the pH of the buffer is 5.0-6.5, preferably 5.5; the buffer includes, but is not limited to, phosphate buffer, acetate buffer, citrate buffer, and succinate buffer, preferably citrate buffer; the concentration of buffer is 10-50 mM, preferably 20 mM.

Further, in order to make the isolated synthesized ADC drugs contain high polymer antibody, a stepwise elution method was employed. The citrate buffer used in the elution process comprises NaCl, and the concentration of NaCl in the elution buffer prepared for the first elution step is 100-140 mM, preferably 110 mM.

Further, in the elution buffer prepared for the second elution step, the buffer comprises 150-200 mM NaCl, preferably 180 mM.

In some embodiments, the binding of the antigen-binding protein to the crosslinker is carried out at a temperature of 20°C to 40°C, the value of which is an integer or a fraction, and the temperatures of which in non-limiting embodiments are preferably 20°C, 22°C, 24°C, 28°C, 32°C, 36°C, more preferably 28°C, and may be 20°C, 21°C, 22°C, 23°C, 24°C, 25°C, 26°C, 27°C, 28°C, 29°C, 30°C, 31°C, 32°C, 33°C, 34°C, 35°C, 36°C, 37°C, 38°C, 39°C, 40°C in certain embodiments.

Further, the binding of the antigen-binding protein to the crosslinker is carried out in a buffer having a pH of 4.0 to 5.5, preferably 4.3; the buffer is preferably an acetate buffer, more preferably an acetate buffer comprising acetonitrile.

Further, for the crosslinker bearing a protecting group, after its binding to the antigen protein, the protecting group of the crosslinker needs to be removed, thereby enabling the crosslinker to bind to the drug; the deprotecting agent may be includes, but is not limited to $NH_2OH{\cdot}HCL$, and the concentration of the deprotecting agent ranges from 10 to 50 mM, preferably 20 mM.

In the method of the present invention, the binding of the antigen-binding protein to the carrier, the binding of the antigen-binding protein to the drug, and the elution of the antigen-binding protein and the drug coupling product are carried out on an ion exchange column.

In the method of the present invention, a rinsing step may be optionally included, and the ion exchange column is rinsing with a buffer containing crosslinker to block the hydrophobicity of the ion exchange filler matrix to prevent the hydrophobic drug from binding to the matrix which leading to a difficult in the elution of ADC drug; if a hydrophilic ion exchange filler matrix, such as agarose, was used, the matrix would react with the lysine residue on the surface of the antibody, thereby blocking the coupling of the drug with the antibody. Therefore, under the strategy of lysine coupling, an ion exchange filler of the hydrophobic matrix is preferred.

## Detailed Discription of the Invention

In order to better understand the present invention, certain technical and scientific terms are specifically defined below. Unless otherwise clearly specified elsewhere in the present invention, all other technical and scientific terms used herein have the meaning commonly understood by those skilled in the art

The term 'humanized antibody' or 'humanized antibodies', also referred to herein as humanization of CDR-grafted antibody or antibodies, which refers to the grafting of a mouse CDR sequences into a human antibody variable region frameworks, ie, antibody produced by different types of human germline antibody framework sequences.

The term 'murine antibody' is an anti-human monoclonal antibody prepared using mice according to the knowledge and skills in the art. During preparation, the test subject is injected with antigen, and then the hybridoma expressing the antibody with desired sequences or functional properties is isolated. The murine antibody or antigen-binding fragment thereof may further comprise a light chain constant region of murine κ, λ chain or variants thereof, or further comprises a heavy chain constant region of murine IgG1, IgG2, IgG3 or IgG4 or variants thereof.

The term 'human antibody' refers to the antibody having amino acid sequence corresponding to the amino acid sequence produced by human or human cell or derived from non-human derived antibody utilizing human antibody library or other human antibody coding sequence. This definition of 'human antibody' specifically excludes humanized antibody comprising non-human antigen-binding residues.

The term 'chimeric antibody' is an antibody obtained by fusing variable region of murine antibody with a constant region of human antibody, which can alleviate the immune response induced by a murine antibody. To construct a chimeric antibody, hybridoma secreting murine-specific monoclonal antibody is first constructed, and then the variable region gene is obtained from the mouse hybridoma cell, and then cloned into the constant region gene of the human antibody for recombinant expression.

The term 'single-chain antibody' refers to an antibody formed by connecting a heavy chain variable region and a light chain variable region through a short peptide of 15 to 20 amino acids. Single-chain antibody is an artificial synthetic antibody that is expressed in *E. coli* using genetic engineering techniques, which contains only one chain of the complete antibody.

The 'antibody' of the present invention refers to any form of antibody that exhibits the desired biological activity. Thus, it is used in the broadest sense and specifically includes, but is not limited to, full length antibody, antibody binding fragments or derivatives. Sources of antibody includes, but is not limited to, monoclonal antibodies, polyclonal antibodies, genetically engineered antibodies (eg, bispecific antibodies).

The term 'Fab fragment' refers to a fragment consisting of a complete light chain and VH and CH1 functional region of a heavy chain. The heavy chain of a Fab molecule cannot form disulfide bridge with another heavy chain molecule.

The term 'Fab' fragment' comprises a light chain and VH and CHI functional region of a heavy chain, and further comprises a region between the CHI and CH2 domains which can form interchain disulfide bridge between two heavy chains of two Fab' fragments to form F(ab')2 molecules.

The term 'F(ab')2 fragment' comprises two light chains and two heavy chains containing a partial constant region between the CHI and CH2 domains such that interchain disulfide bridges are formed between the two heavy chains. Thus, the F(ab')2 fragment consists of two Fab' fragments linked together by disulfide bridges between the two heavy chains.

The term 'scFv fragment' refers to a single-chain variable region (ScFv) produced by genetic engineering

methods, which is a Fv-type fragment comprising VH and VL regions linked together by the flexible polypeptide.

The term 'antigen-binding protein' is a macromolecular compound capable of recognizing and binding to antigen or receptor associated with a target cell. The function of the antigen-binding protein is to present the drug to a target cell population that binds to the antigen-binding protein, including but not limited to protein hormones, lectins, growth factors, antibodies or other molecules capable of binding to cells, preferably antibodies.

The antibody of the present invention refers to monoclonal antibody or mAb, which refers to antibody obtained from a single clonal cell strain; the cell strain is not limited to eukaryotic, prokaryotic or phage clonal cell strain. Monoclonal antibody or antigen-binding fragments can be obtained by recombination using, for example, hybridoma technique, recombinant technique, phage display technique, synthetic techniques (e.g., CDR-grafting), or other prior art.

The antibody of the present invention refers to an immunoglobulin, which is a tetrapeptide chain structure in which two identical heavy chains and two identical light chains are linked by interchain disulfide bridges. The immunoglobulin heavy chain constant region has different amino acid composition and arrangement order, so its antigenicity is also different. Accordingly, immunoglobulins can be classified into five classes, or isotypesnamely IgM, IgD, IgG, IgA and IgE, and the corresponding heavy chains are μ chain, δ chain, γ chain, α chain, and ε chain respectively. The same class of Ig can be divided into different subclasses according to the difference in the amino acid composition of the hinge region and the number and position of heavy chain disulfide bridges. For example, IgG can be classified into IgG1, IgG2, IgG3, and IgG4. Light chains are classified into κ chain or λ chain according to differences between constant regions. In the five classes of Ig, each class of Ig may have κ chain or λ chain.

The sequence of about 110 amino acids near the N-terminus of the antibody heavy and light chains varies greatly and is named the variable region (V region); and the remaining amino acid sequence near the C-terminus is relatively stable and named the constant region (C region). The variable region includes three hypervariable regions (HVR) and four relatively conserved framework regions (FR). The three hypervariable regions determine the specificity of the antibody, also known as the complementarity determining region (CDR). Each of the light chain variable region (LCVR) and the heavy chain variable region (HCVR) consists of three CDR regions and four FR regions, which are sequentially arranged from the amino terminus to the carboxy terminus: FR1, CDR1, FR2, CDR2, FR3, CDR3 and FR4. The three CDR regions of the light chain refer to LCDR1, LCDR2, and LCDR3; and the three CDR regions of the heavy chain refer to HCDR1, HCDR2, and HCDR3. The number and position of CDR amino acid residues of the LCVR region and the HCVR region of the antibody or antigen-binding fragment described in the present invention conform to the known Kabat numbering rules (LCDR1-3, HCDE2-3), or to the kabat and chothia numbering rules (HCDR1).

"Optional" or "optionally" means that the subsequently described event or environment may, but need not, occur, and the description includes occasions where the event or environment occurs or does not occur. For example, "optionally comprising 1-3 antibody heavy chain variable regions" means that a particular sequence of antibody heavy chain variable regions may, but need not, be present.

The term "crosslinker" refers to a class of small molecule compounds, molecules; having two or more reactive ends for a particular group (amino, carboxyl, thiol, etc.) at both ends or in a molecular structure, which may be coupled with two or a plurality of other molecules. Various molecules participating in the reaction are covalently bonded to the crosslinker to form a new molecule.

The term "linker" refers to a chemical module comprising covalent bond or chain of atoms that covalently attaches an antigen-binding protein to a drug. The chemical module is formed by linking a modified antigen-binding protein to a modified moiety of a drug.

The term "conductivity" refers to the ability of an aqueous solution to conduct electrical current between two electrodes. In general, conductivity or specific conductivity is a measure of the conduction current of a substance. In solution, current flows through ion transport. Therefore, as the number of ions present in the aqueous solution increases, the solution will have a higher conductivity. The conductivity is measured in mmhos (mS/cm), and the conductivity of the solution can be changed by changing the ion concentration therein. For example, the concentration of the ionic excipient in the solution can be varied to achieve the desired conductivity.

The term "drug" refers to a toxin (eg, an enzymatically active toxin or a fragment thereof of derived from bacteria, fungi, plant or animal), a chemotherapeutic agent, a growth inhibitor, a tubulin inhibitor, an antibiotic, a radioisotope, and a nucleolytic enzyme and other cytotoxic agents.

The term "toxin" refers to any substance capable of producing a detrimental effect on the growth or proliferation of cells.

The term "tubulin inhibitor" refers to a class of compounds that interfere with the mitotic process of cells by inhibiting the polymerization of tubulin or promoting the polymerization of tubulin, thereby exerting an anti-tumor effect. Non-limiting enbodiments thereof include: maytansinoids, calicheamicin, taxanes, vincristine, colchicine, dolastatin/aluratin, preferably maytansinoids or dolastatin/auristatin.

Maytansinoids are well known in the art and can be isolated from natural sources according to known methods or produced using genetic engineering techniques (Yu et al. The biosynthetic gene cluster of the maytansinoid antitumor agent ansamitocin from Actinosynnema pretiosum. PNAS, 2002, 99: 7968-7973). Maytansinol and analogs of maytansinol can also be prepared synthetically according to known methods. Non-limiting embodiments of maytansinoid alkaloid drug modules include: DM1; DM3; and DM4, as disclosed in patent WO2016127790A1.

Auristatin is a fully synthetic drug with a chemical structural formula, which is relatively easy to modify for optimizing its physical properties and drug properties. The auristatin derivatives used for coupling with the antibody mainly include monomethyl auristatin E (MMAE) and monomethyl auristatin F (MMAF), and the former is a synthetic pentapeptide synthesized by adding a 2-amino-1-phenylpropyl-1-ol at the C-terminus, which derived from natural tubulin polymerase inhibitor dolastatin-10. The inhibitory activity of MMAE against a variety of human tumor cell lines is less than 1 nanomolar. In order to reduce the cytotoxic activity of MMAE, MMAF was developed by adding a phenylalanine to the C-terminus of dolastatin-10. Because of the structural introduction of a carboxyl group, MMAF has poor cell membrane permeability and thus its bioactivity to cells is significantly decreased. However, the inhibitory activity to cells after coupling with antibodies was greatly increased (US7750116).

CPT is an abbreviation for camptothecin, and in the present application CPT is used to denote camptothecin itself or an analog or derivative of camptothecin. The structure of camptothecin and some of its analogs having the number shown and the ring labeled with the letters A-E is provided by the following formula.

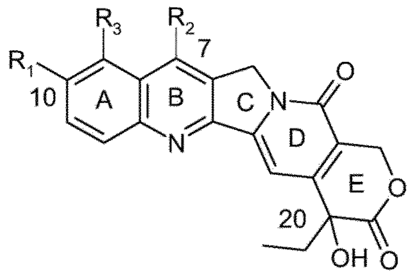

CPT: R1=R2=R3=H
10-hydroxy-CPT: R1=OH; R2=R3=H
Irinotecan (CPT-11) : R1=

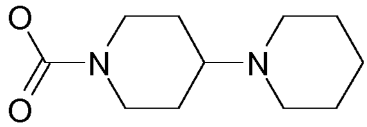

;

R2= Ethyl; R3=H SN-38: R1=OH; R2= Ethyl; R3=H
Topotecan: R1=OH; R2=H; R3=CH-N($CH_3$)$_2$
As used herein, 'antibody-drug conjugate' is referred as 'ADC' interchangeably.

The term 'free mercapto group' in the present invention means a structural group containing a sulfur atom, mainly referring to a mercapto group in a drug or a toxin, a mercapto group exposed after reduction of a disulfide bridge of an antigen-binding protein, or a mercapto group on the lysine or cysteine of an antigen-binding protein lysine or a cysteine.

The term 'thioether bond' refers to a class of structural bonds having the formula -S-.

The term "reducing agent" is a substance that loses electrons or has an electronic deviation in a redox reaction. The reducing agent itself is also an antioxidant in a broad sense, which has reducibility and will be oxidized, and its product is called an oxidation product. In an embodiment of the present invention, the reducing agent is represented by RA, and non-limiting embodiments of the reducing agent include: $H_2$, carbon (C), carbon monoxide (CO), reduced iron powder (Fe), zinc powder (Zn), alkali metal (usually Li, Na, K), other active metals (such as Mg, Al, Ca, La, etc.), stannous chloride ($SnCl_2$), oxalic acid, potassium borohydride ($KBH_4$), sodium borohydride ($NaBH_4$), sodium cyanoborohydride ($NaCNBH_3$), sodium triacetoxyborohydride ((CH3COO) $_3$BHNa), lithium aluminum hydride ($LiAlH_4$), hypophosphorous acid, sodium hypophosphite, sodium thiosulfate ($Na_2S_2O_3$), tris(2-carboxylethyl)phosphine (TCEP), dithiothreitol (DTT), mercaptoethylamine, acetylcysteine (Ac-Cys).

The 'cation exchange carrier' in the present invention means a carrier containing an acidic exchanging group, including a strong acid type cation exchange carrier and a weak acid type cation exchange carrier. The cation exchange carrier comprises a cation exchange resin, a cation exchange membrane, a cation exchange fiber, preferably a cation exchange resin, in the form of a carrier. A strong acid type cation exchange carrier mainly contains strongly acidic reactive group such as a sulfonic acid group (-$SO_3$H), and this ion exchange carrier can exchange all the cations. A weak acid type cation exchange carrier contains weak reactive group such as a carboxyl group (-COOH group), a phosphate group,

etc., and this ion exchange carrier can only exchange cations such as $Ca^{2+}$ and $Mg^{2+}$ in a weak base, while the ions such as Na+ K+, etc., in a strong base cannot be exchanged. Non-limiting emobodiments of strong acid cation exchange resins are: Millpore Fractogel $SO_3$; Millpore Eshmuno S; Millpore Eshmuno CPX; GE CaptoS Impact; GE SP Sepharose Fast Flow; HiTrapTM Capto S ImpAct. Non-limiting embodments of weak acid cation exchange resins are: Millpore EMD COO-; GE CM Sepharose Fast Flow.

The term "alkyl" refers to a saturated aliphatic hydrocarbon radical including straight chain and branched chain groups of 1 to 20 carbon atoms, preferably an alkyl group containing 1 to 12 carbon atoms, more preferably 1 to 10 carbons atoms, most preferably 1 to 6 carbon atoms. Non-limiting embodiments include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-Methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-decyl, 2-methyl-2-ethylhexyl, 2-methyl-3-ethylhexyl, 2,2-diethylpentyl, n-decyl, 3,3-diethylhexyl, 2,2-diethylhexyl, and various branched isomers thereof. The more preferred are lower alkyl groups having 1 to 6 carbon atoms, non-limiting embodiments including methyl, ethyl, n-propyl, isopropyl, n-butyl, isobutyl, tert-butyl, sec-butyl Base, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl,, 2,2-dimethylbutyl, 1,3-dimethylbutyl, 2-ethylbutyl, 2-methylpentyl, 3-methylpentyl, 4-methylpentyl, 2,3-dimethylbutyl and the like. The alkyl group may be substituted or unsubstituted, and when substituted, the substituent may be substituted at any available site for attachment, and one or more of the following groups are preferably selected from the group consisting of an alkyl, alkenyl, alkynyl, alkoxy, alkylthio, alkylamino, halogen, fluorenyl, hydroxy, nitro, cyano, cycloalkyl, heterocycloalkyl, aryl, heteroaryl, ring Alkoxy, heterocycloalkoxy, cycloalkylthio, heterocycloalkylthio, and oxo independently.

The term 'cycloalkyl' refers to a saturated or partially unsaturated monocyclic or polycyclic cyclic hydrocarbon substituent containing 3 to 20 carbon atoms, preferably 3 to 12 carbon atoms, more preferably 3 to 10 carbon atoms, most preferably 3 to 8 carbon atoms. Non-limiting embodiments of monocyclic cycloalkyl groups include cyclopropyl, cyclobutyl, cyclopentyl, cyclopentenyl, cyclohexyl, cyclohexenyl, cyclohexadienyl, cycloheptyl, cycloheptatrienyl, cyclooctyl, etc. Polycyclic cycloalkyl group includes cycloalkyl group of a spiro ring, a fused ring, and a bridged ring.

The term 'heterocyclyl' refers to a saturated or partially unsaturated monocyclic or polycyclic cyclic hydrocarbon substituent containing 3 to 20 ring atoms wherein one or more ring atoms are hetero atoms selected from nitrogen, oxygen or $S(O)_m$ (where m is an integer of 0 to 2), but excluding the ring moiety of -OO-, -OS- or -SS-, and the remaining ring atoms are carbon. It preferably contains 3 to 12 ring atoms, wherein 1 to 4 of which are hetero atoms; more preferably, a cycloalkyl group ring containing 3 to 10 ring atoms. Non-limiting embodiments of monocyclic heterocycl groups include pyrrolidinyl, piperidinyl, piperazinyl, morpholinyl, thiomorpholinyl, homopiperazinyl and the like. Polycyclic heterocycl groups include heterocycl group of spiro ring, fused ring, and bridged ring.

The heterocyclyl ring may be fused to an aryl, heteroaryl or cycloalkyl ring, wherein the ring connected to the parent structure is a heterocyclic group. Non-limiting embodiments of which include:

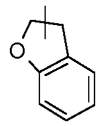 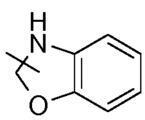 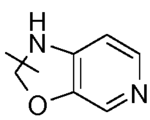 and 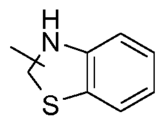,

etc.

The heterocyclic group may be optionally substituted or unsubstituted, and when substituted, the substituent is preferably selected from one or more of the following groups independently: alkyl, alkenyl, alkynyl, alkoxy, alkyl sulphanyl, alkylamino, halogen, fluorenyl, hydroxy, nitro, cyano, cycloalkyl, heterocycloalkyl, aryl, heteroaryl, cycloalkoxy, heterocycloalkoxy, cycloalkylthio, heterocycloalkylthio, and oxo.

The term "aryl" refers to an all-carbon monocyclic or fused polycyclic ring (ie, rings which share adjacent pair of carbon atoms) groups of 6 to 14 carbon atoms having a conjugated π-electron system, preferably 6 to 10 atoms, such as phenyl and naphthyl, preferably phenyl. The aryl ring may be fused to a heteroaryl, heterocyclyl or cycloalkyl ring, wherein the ring to which the parent structure is attached is an aryl ring, non-limiting embodiments of which include:

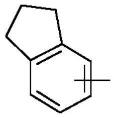 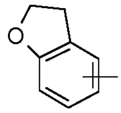 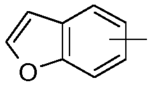 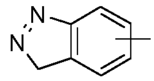 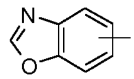 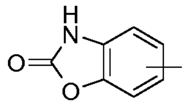 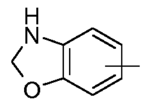

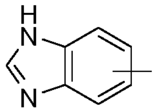 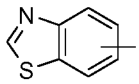 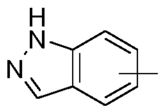 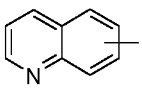 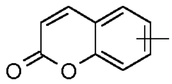 and 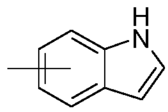 .

The aryl group may be substituted or unsubstituted, and when substituted, the substituent is preferably one or more of the following groups independently selected from alkyl, alkenyl, alkynyl, alkoxy, alkylthio, alkylamino, halogen, fluorenyl, hydroxy, nitro, cyano, cycloalkyl, heterocycloalkyl, aryl, heteroaryl, cycloalkoxy, heterocycloalkoxy, cycloalkylthio, and heterocyclealkylthio.

The term 'heteroaryl' refers to a heteroaromatic system containing 1 to 4 heteroatoms, and 5 to 14 ring atoms, wherein the heteroatoms are selected from the group consisting of oxygen, sulfur and nitrogen. The heteroaryl group is preferably 5 to 10 members, more preferably 5 or 6 members, such as furyl, thienyl, pyridyl, pyrrolyl, N-alkylpyrrolyl, pyrimidinyl, pyrazinyl, imidazolyl, tetrazolyl and the like. The heteroaryl ring may be fused to an aryl, heterocyclic or cycloalkyl ring, wherein the ring connected to the parent structure is a heteroaryl ring, non-limiting embodiments of which include:

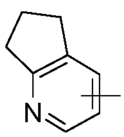 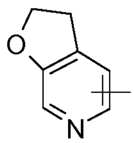 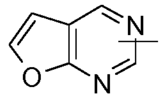 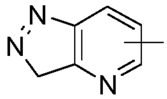 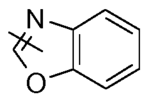 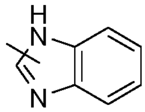 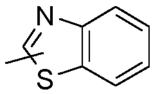

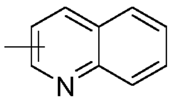 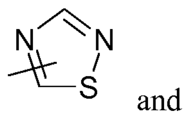 and 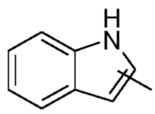 .

The heteroaryl group may be optionally substituted or unsubstituted, and when substituted, the substituent is preferably one or more of the following groups independently selected from alkyl, alkenyl, alkynyl, alkoxy, alkylthio, alkylamino, halogen, fluorenyl, hydroxy, nitro, cyano, cycloalkyl, heterocycloalkyl, aryl, heteroaryl, cycloalkoxy, heterocycloalkoxy, cycloalkylthio, and heterocycloalkylthio.

The term 'alkoxy' refers to -O-(alkyl) and -O- (unsubstituted cycloalkyl), wherein the alkyl or the cycloalkyl is as defined above. Non-limiting embodiments of alkoxy groups include: methoxy, ethoxy, propoxy, butoxy, cyclopropoxy, cyclobutoxy, cyclopentyloxy, or cyclohexyloxy. The alkoxy group may be optionally substituted or unsubstituted, and when substituted, the substituent is preferably one or more of the following groups independently selected from alkyl, alkenyl, alkynyl, alkoxy, alkylthio, alkylamino, halogen, fluorenyl, hydroxy, nitro, cyano, cycloalkyl, heterocycloalkyl, aryl, heteroaryl, cycloalkoxy, heterocycloalkoxy, cycloalkylthio, and heterocycloalkylthio.

The term 'alkylamino' refers to -N-(alkyl) and -N- (unsubstituted cycloalkyl), wherein the alkyl or the cycloalkyl is as defined above. Non-limiting embodiments of alkylamino groups include: methylamino, ethylamino, propylamino, butylamino, cyclopropylamino, cyclobutylamino, cyclopentylamino, or cyclohexylamino. The alkylamino group may be optionally substituted or unsubstituted, and when substituted, the substituent is preferably one or more of the following groups independently selected from alkyl, alkenyl, alkynyl, alkoxy, alkanethio, alkylamino, halogen, fluorenyl, hydroxy, nitro, cyano, cycloalkyl, heterocycloalkyl, aryl, heteroaryl, cycloalkoxy, heterocycloalkoxy, cycloalkylthio, and heterocycloalkylthio.

The term 'bond' refers to a covalent bond represented by '-'.

The term 'hydroxy' refers to an -OH group.

The term 'halogen' means fluoro, chloro, bromo or iodo.

The term 'carboxylate group' refers to -C(O)O(alkyl) or (cycloalkyl) wherein the alkyl or the cycloalkyl is as defined above. 'Optional' or 'optionally' means that the subsequently described event or environment may, but need not, occur, and the description includes occasions where the event or environment occurs or does not occur. For example, 'heterocyclic group optionally substituted by an alkyl group' means that an alkyl group may be, but not necessarily,

present, and the description includes occasion where the heterocyclic group is substituted with an alkyl group and the occasion where the heterocyclic group is not substituted with an alkyl group.

'Substituted' refers to one or more hydrogen atoms in the group, preferably up to 5, more preferably 1 to 3 hydrogen atoms, independently of each other, substituted by a corresponding number of substituents. It goes without saying that the substituents are only in their possible chemical positions, and those skilled in the art will be able to determine (by experiment or theory) substitutions that may or may not be possible without undue effort. For example, an amino group or a hydroxyl group having a free hydrogen may be unstable when combined with a carbon atom having an unsaturated (e.g., olefinic) bond.

Abbreviation

MMAE=monomethyl auristatin E (MW718)
MMAF=A variant of auristatin E (MMAE) with phenylalanine at the C-terminus of the drug (MW731.5)
DM1=N(2')-deacetyl-N(2')-(3-mercapto-1-oxypropyl)-maytansine
DM3=N(2')-deacetyl-N2-(4-mercapto-1-oxopentyl)-maytansine
DM4=N(2')-deacetyl-N2-(4-mercapto-4-methyl-1-oxopentyl)-maytansine
SMCC=Succinimidyl 4-(N-maleimidomethyl)cyclohexane-1-carboxylate
LC-SMCC=Succinimidyl-4-(N-maleimidomethyl)cyclohexane-1-carboxy-(6-amidocaproate)
KMUA=κ-maleimide undecanoic acid N-succinimidyl ester
GMBS=y-maleimide butyric acid N-succinimidyl ester
EMCS=ε-maleimidocaproic acid N-hydroxysuccinimide ester
MBS=m-maleimidobenzoyl-N-hydroxysuccinimide ester
AMAS=N-(α-maleimidoacetate)-succinimidyl ester
SMPH=succinimidyl-6-(β-maleimidopropionamido)hexanoate
SMPB=N-succinimidyl 4-(p-maleimidophenyl)-butyrate
PMPI=N-(p-maleimidophenyl)isocyanate
SIAB=N-succinimidyl-4-(iodoacetyl)-aminobenzoate
SIA=N-succinimidyl iodoacetate
SBA=N-succinimidyl bromoacetate
SBAP=N-succinimidyl 3-(bromoacetylamino)propionate

Method for synthesizing the compound of the present invention

In order to accomplish the objectives of the present invention, the present invention adopts the following technical solutions:

Solution 1

The invention also provides a preparation method of the general formula ADC-1, wherein the steps include the a step (as in the embodiment 2), the b step (as in the embodiment 4), the c step (as in the embodiment 5) in the above step 1), and step 2) and step 3) (as in embodiment 6); details are as follows:

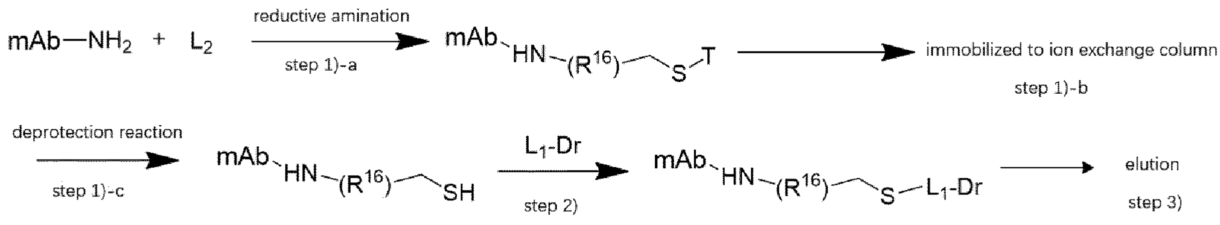


ADC-1

The mAbs described herein are the antigen-binding proteins of the present invention, and the definitions of $L_1$-Dr and $L_2$ are as defined in the general formula ($L_2$), and the formula ($L_1$-Dr).

Non-limiting embodiments of the general formula ADC-1 prepared by the present invention are as follows:

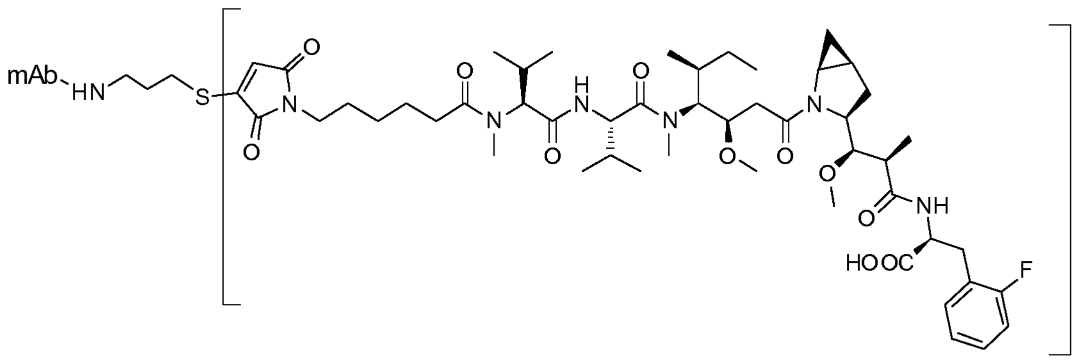

The mAb described herein is the EGFR antibody described in Embodiment 2 and the c-Met antibody described in Embodiment 11.

Solution 2

The present invention also provides a preparation method of the general formula ADC-2 (see illustrative embodiment such as Embodiment 9), the steps including the A step, the B step in the above step 1), and the step 2) and the step 3); details are as follows:

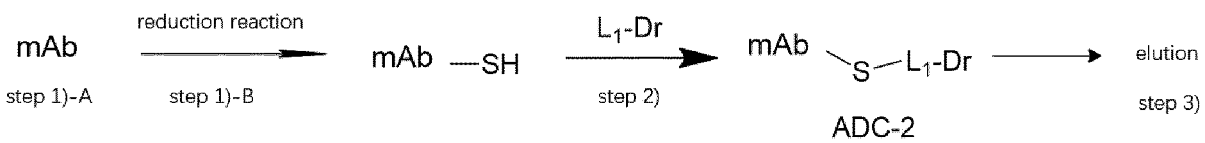


ADC-2

The mAb is the antigen-binding protein of the present invention, and the antigen-binding protein is immobilized on the ion carrier by the step A in the step 1), and the reduction reaction is further carried out to react with the drug; the definition of $L_1$-Dr is as described in the general formula ($L_1$-Dr).

Non-limiting embodiment of the general formula ADC-2 prepared by the present invention are as follows:

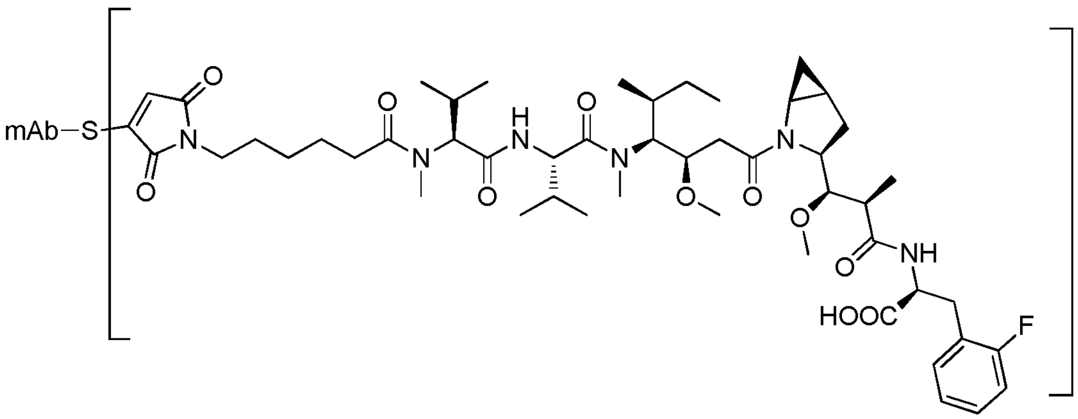

The mAb described herein is the EGFR antibody described in Embodiment 9 and the c-Met antibody described in Embodiment 11.

The beneficial effects of the invention are:

1. In the present invention, immobilization of biomolecules is realized by an electrostatic interaction between an antibody biomolecule and an ion exchange resin, thereby avoiding aggregation of biomolecules caused by mutual collision during the coupling process.
2. The process steps of the invention are simple and convenient to operate. It realizes programmatic control, avoids human error, and improves production efficiency.
3. By immobilizing biomolecules such as antibodies, zero retention of organic solvents is achieved, and side effects

of drugs are reduced.

4. By optimizing elution conditions, the DAR value (drug antibody binding ratio) can be effectively controlled within the required range, mean while the antibody-conjugated drug containing the polymer can be separated and removed.

5. The production process conditions are mild, avoiding mechanical damage to biomolecules such as antibodies, and ensuring the integrity of the antibody.

6. The concentration and usage of toxic small molecules and the toxicity to human body and environmental pollution during the operation can by reduced by the present process.

7. The cation purification step in the antibody purification process can be eliminated and the purification efficiency can be improved.

8.The synthesis of ADC drugs by means of ion exchange carrier reduces the production cost, while handling a large amount of sample. It is expected that each liter of filler can handle no less than 50 g of biomolecules such as antibodies, which is helpful for batch scale-up production.

## Brief description of the drawings

Figure 1. Reaction equation for antibody and crosslinker;

Figure 2. Effect of molar ratio of crosslinker to antibody on LAR value;

Figure 3. Removal reaction equation for crosslinker teminal protecting group;

Figure 4. Flow diagram of the ligation of toxin and the elution of antibody-drug conjugate;

Figure 5. Stepwise elution chromatogram of EGFR antibody-drug conjugate;

Figure 6. Stepwise elution chromatogram of ADC obtained by EGFR antibody interchain disulfide bridge coupling;

Figure 7. SEC analysis results of eluted antibody-drug conjugates using lysine coupling; (A) analysis chart of antibody-crosslinker agent; (B) analysis chart of elution peak A; (C) analysis chart of eluntion peak B; (D) analysis chart of elution peak C;

Figure 8. Mass spectrometry analysis of antibody binding to crosslinker;

Figure 9. Mass spectrometry analysis of EGFR antibody-drug conjugates;

Figure 10. HIC analysis of the interchain disulfide bridge coupling of EGFR antibody;

Figure 11. Analysis of ADC drug coupling results of c-Met antibody; (A) Mass spectrometry of lysine coupling results; (B) HIC diagram of interchain disulfide bridge reductive coupling.

## Detailed description of the preferred embodiment

The present disclosure is not to be limited in scope by the specific embodiments described which are intended as illustrations of individual aspects of the disclosure, and the spirit and scope of the invention is not limited thereto. Unless otherwise specified, the experimental methods in the embodiments of the present invention are selected generally according to the conventional conditions which are favorable for production; or according to the conditions recommended by the manufacturer of the raw material or the commodity. Unless otherwise specified, the reagents used herein are commercially available.

## Embodiment 1. Construction and expression of EGFR antibody

The EGFR antibody mAb001 is an IgG1-YTE modified imotuzumab variant obtained by point mutation of nimotuzumab.

Primers were designed to PCR constructing the antibody VH/VK gene fragment, and then homologously recombined with the expression carrier pHr (with signal peptide and constant region gene (CH1-FC/CL) fragment) to construct the full-length antibody expression carrier VH-CH1-FC-pHr/VK-CL-pHr. The original form of the plasmid can express IgG1, and IgG1-YTE antibody comprising triple site mutations, i.e. M258Y/S260T/T262E (YTE), was obtained by point mutation. The final EGFR antibody mAb001 sequence is set forth in SEQ ID NO: 1 and SEQ ID NO: 2. After the plasmid was verified by sequencing and isolated by a well-known method in the art, and 293 cell transient expression was performed, thereby obtaining the culture supernatant containing the antibody protein of interest for isolation and purification.

Antibody sequence of mAb001 is as follows:

amino acid sequence of mAb001 light chain: SEQ ID NO: 1

DIQMTQSPSSLSASVGDRVTITCRSSQNIVHSNGNTYLDWYQQTPGKAPKLLIYKV SNRFSGVPSRFSGSGSGTDFTFTISSLQPEDIATYYCFQYSHVPWTFGQGTKLQITRTVA APSVFIFPPSDEQLKSGTASVVCLLNNFYPREAKVQWKVDNALQSGNSQESVTEQDSK DSTYSLSSTLTLSKADYEKHKVYACEVTHQGLSSPVTKSFNRGEC

amino acid sequence of mAb001 heavy chain: SEQ ID NO:2

QVQLQQSGAEVKKPGSSVKVSCKASGYTFTNYYIYWVRQAPGQGLEWIGGINPT SGGSNFNEKFKTRVTITADESSTTAYMELSSLRSEDTAFYFCTRQGLWFDSDGRGFDFW GQGTTVTVSSASTKGPSVFPLAPSSKSTSGGTAALGCLVKDYFPEPVTVSWNSGALTSG

VHTFPAVLQSSGLYSLSSVVTVPSSSLGTQTYICNVNHKPSNTKVDKKVEPKSCDKTHT CPPCPAPELLGGPSVFLFPPKPKDTLYITREPEVTCVVVDVSHEDPEVKFNWYVDGVE VHNAKTKPREEQYNSTYRVVSVLTVLHQDWLNGKEYKCKVSNKALPAPIEKTISKAK GQPREPQVYTLPPSRDELTKNQVSLTCLVKGFYPSDIAVEWESNGQPENNYKTTPPVLD SDGSFFLYSKLTVDKSRWQQGNVFSCSVMHEALHNHYTQKSLSLSPGK

Note: Double underlined represents the YTE mutation sites.
purification and analysis of mAb001 antibody:
The above cell culture supernatant was centrifuged at high speed to remove impurities, and then subjected to Protein A column affinity chromatography. Rinsed the column with PBS until the A280 reading dropped to baseline. The protein of interest was eluted with 100 mM sodium acetate pH 3.0 and neutralized with 1 M Tris-HCl. The eluted sample was appropriately concentrated, and further purified by molecular sieve using gel chromatography column Superdex 200 (GE) which was balanced by a PBS, and the samples of the absorption peak having antibody monomerswere collected and pooled. The sample could be concentrated or the sample buffer can be replaced by ultrafiltration methods well known in the art to obtain a final sample with suitable concentration.

**Embodiment 2. Reductive amination reaction of monoclonal antibody EGFR antibody and crosslinker**

On the basis of a mechanism of lysine coupling, the stock solution of 20 mg/ml monoclonal antibody mAb001 was replaced with a modification buffer (100 mM acetic acid + 10% acetonitrile, pH 4.3), and 0.65-1.7 mM bifunctional crosslinker 3-acetyl mercapto (ATPPA) and 25 mM reducing agent sodium cyanoborohydride ($NaBH_3CN$) were added, thereby modifying the lysine at a temperature of 24-36°C with stirring at 100 rpm for 2 hours. The amino group ($-NH_2$) on the monoclonal antibody was reductively aminated with the aldehyde group at the end of the crosslinker to form the intermediate I, and the reaction equation was shown in FIG. 1. The LAR value differred from the amount of mAb-crosslinker produced under reaction conditions of different temperature, as shown in Table 1, in which the molar ratio of the crosslinker to the antibody is 6. The free crosslinker was removed by ultrafiltration (UF) system and the buffer of Intermediate I was replaced with a new buffer (20 mM phosphate buffer, pH 6.3), and stored until use. FIG. 2 shows the optimization of the molar ratio of the crosslinker to the monoclonal antibody. When the ratio of the crosslinker to the monoclonal antibody was controlled at 8:1 at a temperature of 28°C for 2 hours, the coupling ratio of the crosslinker-monoclonal antibody ratio (LAR) in the product can be controlled at about 3.5.

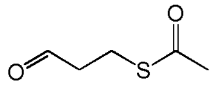

3-acetyl mercapto (ATPPA)

Table 1 Effect of different temperatures on the binding of mAb001 and crosslinker

| Temperature (°C) | LAR value | mAb content (%) |
|---|---|---|
| 24 | 1.68 | 97.96 |
| 28 | 1.84 | 97.71 |
| 32 | 2.04 | 96.72 |
| 36 | 2.23 | 96.21 |

**Embodiment 3. Crosslinker rinse for ion exchange column**

In the process of synthesizing ADC by lysine coupling strategy, 1 ml of cation exchange resin Fractogel®$SO_3$ (M) from MercK Millipore was used as a carrier to synthesize ADC. A strong hydrophobic matrix was blocked with crosslinker according to the following steps.

1) Equilibration: 5 column volumes (CV) of 100 mM acetate buffer (5% acetonitrile, pH 4.3) flowed through 1 ml of the cation exchange column Millipore SO3 (M) at a flow rate of 0.2 ml/min.
2) Rinse: 650 mM 3-acetyl mercapto (ATPPA) solution was prepared with the above described acetate buffer for equilibration, and the column was rinsed and equilibrated at a flow rate of 20 CV at 0.2 ml/min.

**Embodiment 4. Binding of antibody linked to crosslinker and ion exchange column**

1) Equilibration: Using a strong cation ($-SO_3$) exchangeable resin as the medium, 5 CV of the equilibration buffer was used to rinse the resin at a flow rate of 0.2 ml/min.
2) Loading: Controlling the conductivity of the intermediate I solution within 5 mS/cm, adjusting the pH to 6.3 with 1 M citric acid and 1 M Tris, flowing the intermediate I solution through the ion exchange column at a flow rate of 0.2 ml/min, and the capacity of which was controlled at 10-50 mg/ml.
3) Rinsing: Rinsing the column with 3 CV of equilibration buffer.
4) 2nd rinsing: Thoroughly removing the ions accumulated on the ion exchange column with 3 CV of reaction buffer (100 mM acetate buffer + 5% acetonitrile, pH 4.3) at a flow rate of 0.2 ml/min.
5) 3rd rinsing: Removing acetonitrile with 5 CV of equilibration buffer at a flow rate of 0.2 ml/min.

**Embodiment 5. Deprotection at the terminal of the crosslinker**

Intermediate I bound to the ion exchange column contains crosslinker with terminal protecting group, and the terminal protecting group of which needed to be converted to free mercapto group with deprotecting agent hydroxylamine hydrochloride ($NH_2OH{\cdot}HCL$) for facilitating the subsequent binding of intermediate I to toxin. A 20 mM hydroxylamine hydrochloride solution was prepared with the equilibration buffer, and the column was rinsed with 12 CV of hydroxylamine hydrochloride solution at a flow rate of 0.2 ml/min to completely reduce the sulfhydryl group at the terminal of the crosslinker to form intermediate II. The reaction equation is shown in FIG. 3. After the reaction was terminated, the column was rinsed directly with 5 CV of equilibration buffer at a flow rate of 0.2 ml/min.

**Embodiment 6. Coupling of antibody and toxin**

The structure of the toxin used in this experiment is as follows:

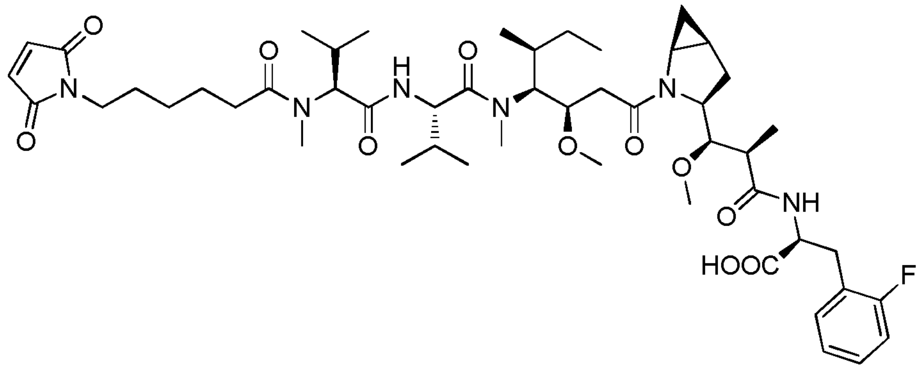

Toxins can be prepared according to the method of patent application WO2016127790A1. In the process of coupling toxin, a new reaction buffer (20 mM phosphate buffer + 5% acetonitrile, pH 6.3) is used to prepare a 40 CV of 10 mM toxin solution. See Figure 4 for the whole process.

1) Equilibration: The intermediate II bound ion exchange column was equilibrated with a total of 5 CV new buffer (20 mM phosphate buffer + 5% acetonitrile, pH 6.3) at a flow rate of 5 CV at a flow rate of 0.2 ml/min.
2) Loading: 30-50 CV of 10 mM toxin was flowed through the ion exchange column at a flow rate of 0.2 ml/min and reacted at room temperature.
3) Rinsing: Unbound toxins in the reaction was rinsed with 5 CV of buffer (20 mM phosphate buffer + 5% acetonitrile, pH 6.3) at a flow rate of 0.2 ml/min.
4) Secondary rinsing: Rinsing residual acetonitrile with 5 CV of equilibration buffer (100 mM phosphate buffer, pH 6.3) at a flow rate of 0.2 ml/min.
5) Elution: Elution was carried out with 15 CV of elution buffer (20 mM citric acid + 110 mM NaCl, pH 5.5) at a flow rate of 0.2 ml/min.
6) Secondary elution: another 15 CV of elution buffer (20 mM citric acid + 180 mM NaCl, pH 5.5) was used to elute the polymer that may be produced at a controlled flow rate of 0.2 ml/min. The entire elution chromatography is shown in Figure 5, and the relevant data analysis is shown in Table 2.
7) Regeneration: The ion exchange column was regenerated with 5 CV of 1 M NaOH at a flow rate of 0.2 ml/min.

As a result, the illustrated ADC structure obtained by the present invention is as follows:

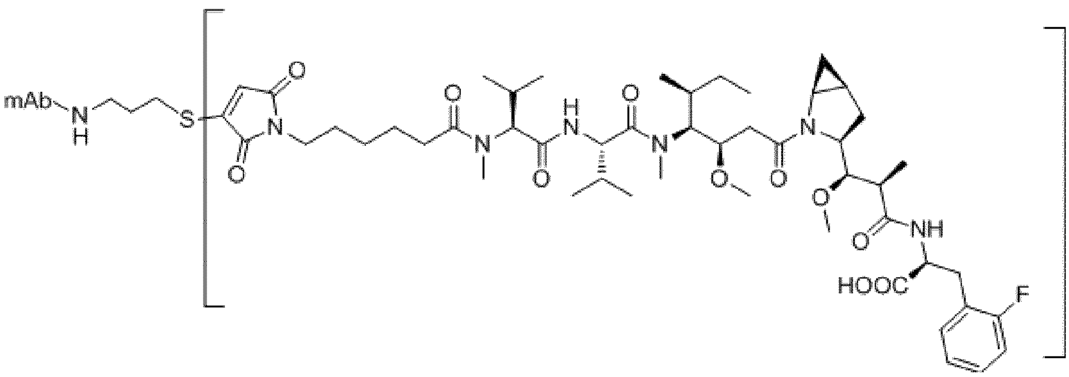

Table 2 Characterization of eluting peaks at different stages

| Sample | LAR/ DAR① | Polymer Content② (%) | mAb② (%) | Fragment Content② (%) | Volume (ml) | Concentration (mg/ml) | pH | Yield (%) |
|---|---|---|---|---|---|---|---|---|
| Lysine Coupling Strategy | | | | | | | | |
| mAb-crosslinker | 3.58 | 5.28 | 94.39 | 0.33 | 22 | 1.8 | 6.3 | 100 |

(continued)

| Sample | LAR/ DAR① | Polymer Content② (%) | mAb② (%) | Fragment Content② (%) | Volume (ml) | Concentration (mg/ml) | pH | Yield (%) |
|---|---|---|---|---|---|---|---|---|
| Lysine Coupling Strategy | | | | | | | | |
| Peak A | 2.59 | 3.23 | 96.77 | 0 | 8.1 | 3.73 | 5.5 | 75.5 |
| Peak B | 2.88 | 44.89 | 55.11 | 0 | 3.3 | 0.73 | 5.5 | 6.08 |
| Peak C | 3.26 | 56.07 | 53.93 | 0 | 1.0 | 0.9 | 5.5 | 2.27 |
| Interchain Disulfide Bridge Coupling Strategy | | | | | | | | |
| mAb | 0 | 2.37 | 96.93 | 0.7 | 10.6 | 1.88 | 7.4 | 100 |
| Peak A | 3.34 | 0.6 | 98.94 | 0.46 | 3.57 | 2.23 | 5.5 | 39.8 |
| Peak B | 4.61 | 21.5 | 76.56 | 1.94 | 2 | 0.78 | 5.5 | 7.8 |

Note: ① For the lysine coupling strategy, the data is derived from the analysis results of MS; for the coupling strategy of interchain disulfide bridge, the data is derived from the analysis results of HIC; ② The data is derived from the analysis results of SEC

**Embodiment 7. Size exclusion chromatography (SEC) analysis method**

In the preparation of ADC drugs, only the conjugates of monoclonal antibodies and toxins are truly curative. To analyze the content of active ingredients in ADC drugs, Waters X Bridge® BEH SEC column (PN: 186007640; SN: 01143613316121; Size: 7.8×300 mm, 200 Å, 3.5 μm), prepacked column (PN: 186007638; SN: 01093616216101; Size: 7.8×30 mm, 200 Å, 3.5 μm) was used, with a 100 mM of flowability , a pH 6.7 phosphate buffer containing 20 mM sodium sulfate, and a 3% (v/v) isopropanol. SEC analysis was performed on the different elution peaks in the chromatographic results. 100 μg of protein was injected and the absorption peak at 280 nm was measured at a flow rate of 0.5 ml/min. The results are shown in Fig. 7.

**Embodiment 8. Mass spectrometry analysis of drug toxin-antibody coupling ratio (DAR) of small molecule drug toxin to antibody**

The ADC drug (peak A) eluted from the above cation exchange column was treated by Waters Xevo G2-XS Q-TOF (Waters Corporation, Milford, Massachusetts) in a positive ion ESI mode in the range of 500-5000 m/z, and mass spectrometry data of deglycosylated ADC molecules was obtained. The desolvation gas temperature was 450°C, the gas flow rate was 800 L/hr, the ion source temperature was 120°C, and the capillary voltage was 3000 V, respectively. The raw data was converted to a zero-charge mass spectrometry using the highest entropy deconvolution algorithm in UNIFI software version 1.8. The molecular weight of mAb001 antibody, the crosslinker, and the toxin are 147,458 Da, 132 Da and 955 Da, respectively. The loading amount of the deglycosylated ADC molecule is 1 μg, and the drug toxin-antibody coupling ratio (DAR) is calculated as:

$$\mathrm{DAR} = \frac{\sum_0^n nV_n}{\sum_0^n \mathrm{V_n}}$$

Note: DAR: coupling ratio of toxin to antibody; $V_n$: peak area of antibody coupled with n drug molecules; n=0, 1, 2, 3 ... (n≥0)

The ratio of drug toxin-antibody coupling ratio (DAR) is critical for the efficacy of ADC drugs, and the crosslinker-monoclonal antibody ratio (LAR) directly affects the subsequent DAR. Figure 8 is a mass spectrogram of EGFR antibody coupled with crosslinker with a LAR value of 3.58, Figure 9 is a mass spectrometric analysis of ADC drug of EGFR antibody with a DAR value of 2.59.

**Embodiment 9. Reduction reaction of interchain disulfide bridge of monoclonal EGFR antibody**

The coupling strategy for interchain disulfide bridge differred from the lysine coupling mechanism. Before the EGFR antibody bound to the carrier, the disulfide bridge is unbroken, and the hydrophilic carrier matrix would not interact with the disulfide bridge. Therefore, 1 ml of GE's cation exchange resin HiTrapTM Capto S ImpAct is used as a immobilized carrier, and the matrix of the which is hydrophilic agarose.

The column was equilibrated with 20 mM pH 6.3 phosphate buffer containing 2 mM EDTA at a flow rate of 0.2 ml/min. Then 60 CV of EGFR monoclonal antibody sample was loaded at the same flow rate, and the loading was controlled at 20 mg. Equilibration buffer was used to continue rinse and remove unbound monoclonal antibody or impurities. Subsequently the ion exchange column was transferred to a 40°C insulation interlayer (to ensure that the reducing agent TCEP and EGFR antibodies are reacted at 40°C). 24 CV of the reducing agent TCEP having 6 times molar concentration of the antibody was dissolved in the equilibration buffer, and slowly flowed through the column at a flow rate of 0.2 ml/min. Immediately after the reduction reaction, the ion exchange column was taken out of the insulation interlayer and placed at room temperature (20-25°C, Industrial routine production with 25°C), and drugs was added and contacted with the immobilized antigen-binding protein, ensuring that the reaction of the drug with the reduced mercapto group was carried out at room temperature. The remaining reducing agent does not need to be rinsed and is directly rinsed and removed at the next step of coupling with the drug.

The structure of the ADC obtained by this method is as follows. The binding to the toxin and the elution of the product are similar to those of Embodiment 6. The elution chromatography is shown in Fig. 6, and the relevant data analysis is shown in Table 2.

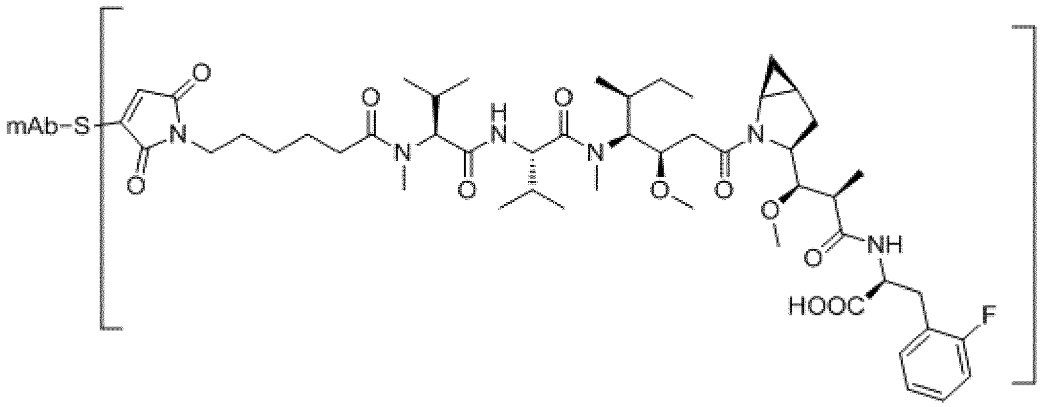

**Embodiment 10. Hydrophobic interaction chromatography (HIC)**

Since the coupling strategy of the disulfide bridge requires break the disulfide bridge first between the heavy chains or between heavy chain and light chain of the EGFR antibody, a series of mass spectrometry peaks of heavy chains and light chains will be obtained when analyzing coupling value of the drug by mass spectrometry, which makes analysis of DAR value difficult. Hence the DAR value was determined by HIC analysis herein according to the strong hydrophobicity of the antibody binding drug.

Hydrophobic chromatography was performed using a TOSOH TSK-butyl NPR column with a linear gradient of 0-100% buffer A and B within 12 min at a flow rate of 0.5 ml/min. Wherein buffer A contains 1.5 M ammonium acetate, and 25 mM sodium phosphate, pH 6.95, and buffer B contains 25 mM sodium phosphate, 25% IPA, pH 6.95. The drug-antibody coupling ratio of the conjugate was determined by the integrating absorbance at 280 nm of the elution peak area. Figure 10 shows the results of random coupling of disulfide bridges of EGFR antibodies (DAR = 3.34). The method used herein has certain versatility and is not limited by the types of antibodies.

**Embodiment 11. Preparation of c-Met antibody drug Conjugate**

In order to verify the versatility of the present invention in the field of monoclonal antibody application, another antibody: c-Met antibody was selected for the preparation of the ADC drug. Two coupling methods, lysine coupling and interchain disulfide bridge coupling strategy, were also employed, and the specific experimental methods and toxins used herein are similar to the EGFR antibody drug conjugates prepared in the above embodiments.

The c-Met antibody mAb002 is a humanized monoclonal antibody prepared according to the method of CN106188293A.

Antibody sequence of mAb002 is as follows:

mAb002 light chain amino acid sequence: SEQ ID NO:3

DIVLTQSPDSLAVSLGERATINCRADKSVSTSTYNYLHWYQQKPGQPPKLLIYLAS NLASGVPDRFSGSGSGTDFTLTISSLQAEDVAVYYCQHSRDLPPTFGQGTKLEIKRTVAA PSVFIFPPSDEQLKSGTASVVCLLNNFYPREAKVQWKVDNALQSGNSQESVTEQDSKD STYSLSSTLTLSKADYEKHKVYACEVTHQGLSSPVTKSFNRGEC

mAb002 heavy chain amino acid sequence: SEQ ID NO:4

QVQLVESGGGVVQPGRSLRLSCAASGFSLSNYGVHWVRQAPGKGLEWLAVIWS GGSTNYAAAFVSRLTISKDNSKNTVYLQMNSLRAEDTAVYYCARNHDNPYNYAMDY WGQGTTVTVSSASTKGPSVFPLAPCSRSTSESTAALGCLVKDYFPEPVTVSWNSGALTS GVHTFPAVLQSSGLYSLSSVVTVPSSNFGTQTYTCNVDHKPSNTKVDKTVERKCCVEC PPCPAPPVAGPSVFLFPPKPKDTLMISRTPEVTCVVVDVSHEDPEVQFNWYVDGVEVH NAKTKPREEQFNSTFRVVSVLTVVHQDWLNGKEYKCKVSNKGLPAPIEKTISKTKGQP REPQVYTLPPSREEMTKNQVSLTCLVKGFYPSDIAVEWESNGQPENNYKTTPPMLDSD GSFFLYSKLTVDKSRWQQGNVFSCSVMHEALHNHYTQKSLSLSPGK

The results of lysine coupling of the c-Met antibody drug conjugate were analyzed using MS, as shown in Figure 11A. The structure of the c-Met antibody ADC obtained by the lysine coupling method is as follows:

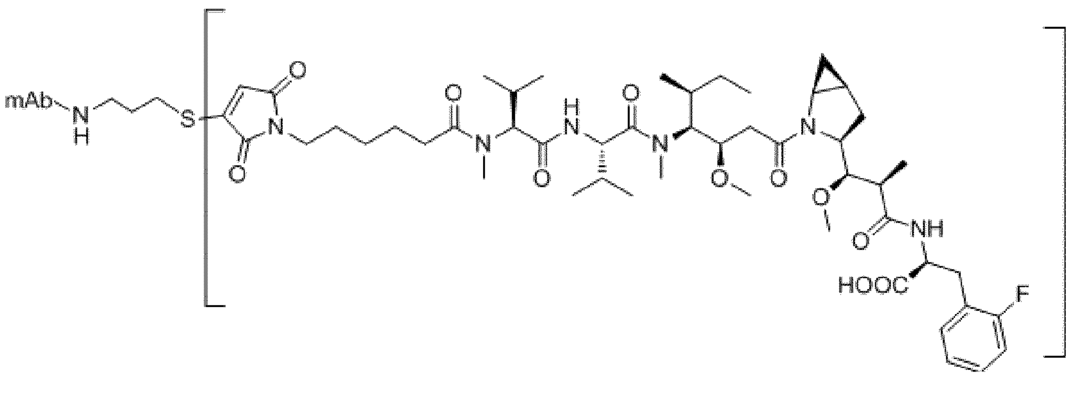

During the coupling of interchain disulfide bridge, the reducing agent was replaced by DTT from the original TCEP, and the concentration of DTT was 8 times of the molar concentration of c-Met antibody. The coupling step and other parameters are similar to those of EGFR antibody. The HIC analysis of the coupled product is shown in Figure 11B. The disulfide bridge between the c-Met antibody chains is almost entirely broke, and the DAR of the ADC drug is 4.85. The ADC drug structure of the interchain disulfide bridge reductive coupling c-Met antibody is as follows:

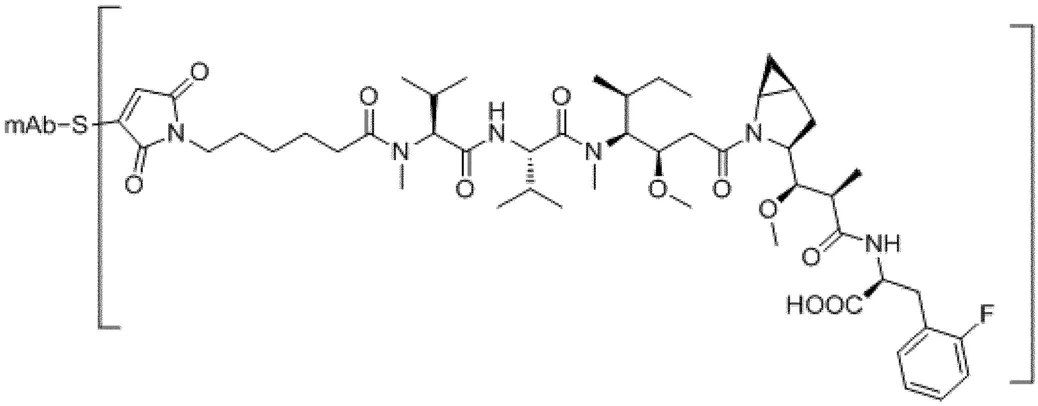

## Claims

1. A method for preparing an antigen-binding protein-drug conjugate, wherein the method comprises the following steps:

    1) immobilizing an antigen-binding protein on an ion exchange carrier to form an immobilized antigen-binding protein;
    2) contacting the immobilized antigen-binding protein with a drug to form an antigen-binding protein-drug conjugate;
    3) eluting the antigen-binding protein-drug conjugate from the ion exchange carrier.

2. The method of claim 1, wherein said ion exchange carrier is selected from the group consisting of ion exchange resins, ion exchange membranes, ion exchange fibers, preferably ion exchange resins.

3. The method of claims 1 or 2, wherein the ion exchange carrier is a cation exchange carrier, and the cation exchange carrier preferably contains a strongly acidic reaction ligand, and the strongly acidic reaction ligand is preferably a sulfonate.

4. The method of any one of claims 1 to 3, wherein the immobilized antigen-binding protein in step 2) is coupled to a drug, and the coupling reaction is that the antigen-binding protein and the drug are linked through an interaction between a nucleophilic group and an electrophilic group;
    wherein the nucleophilic group is optionally from the antigen-binding protein or the drug, preferably from the antigen-binding protein;
    wherein the electrophilic group is optionally from the antigen-binding protein or the drug, preferably from the drug.

5. The method of any one of claims 1 to 4, wherein the nucleophilic group is selected from the group consisting of a mercapto, a hydroxyl group, an amino group, a hydrazide, an oxime, a hydrazine, a thiosemicarbazone, a hydrazine carboxylate, and an aryl hydrazide group, provided that:

    when the nucleophilic group is from the drug, the nucleophilic group is preferably a mercapto group;
    when the nucleophilic group is from the antigen-binding protein, the nucleophilic group is preferably an amino group, a mercapto group, or a hydroxyl group; and the amino group is more preferably an N-terminal amino group, a side chain amino group, or an amino group of a saccharide in a glycosylated antigen-binding protein; the hydroxyl group is more preferably a hydroxyl group of a saccharide in a glycosylation antigen-binding protein, and the mercapto group is more preferably a thiol side chain , and most preferably a thiol side chain of a cysteine.

6. The method of any one of claims 1 to 5, wherein the antigen-binding protein-drug conjugate is coupled by a linker selected from a cleavable linker or an uncleavable linker.

7. The method of any one of claims 1 to 6, wherein the electrophilic group is selected from the group consisting of an active ester, a hydrocarbyl halide, a benzyl halide, an aldehyde, a ketone, a carboxyl group, and a maleimide group, provided that:

    when the electrophilic group is from the antigen-binding protein, the electrophilic group is preferably derived from an aldehyde, a ketone, a carboxyl group, and a maleimide group, more preferably a maleimide group;
    when the electrophilic group is from the drug, the electrophilic group is preferably an active ester, a hydrocarbyl halide, a maleimide group, more preferably a maleimide group; the active ester is preferably an NHS ester, an HOBt ester, a halo formate, an acid halide, and the hydrocarbyl halide is preferably a haloacetamide.

8. The method of any one of claims 1 to 7, wherein the electrophilic group is derived from the drug itself or from a modification of the drug.

9. The method of any one of claims 1 to 8, wherein the immobilized antigen-binding protein is coupled to a drug selected from the group consisting of lysine coupling, light-and-heavy interchain reductive disulfide bridge coupling, site-directed coupling, preferably lysine coupling, light-and-heavy chain reductive disulfide bridge coupling.

**10.** The method of any one of claims 1 to 9, wherein the antigen-binding protein in step 1) is optionally selected from a modified antigen-binding protein or an unmodified antigen-binding protein, preferably a modified antigen-binding protein; the modified antigen-binding protein is optionally an antigen-binding protein that binds a chemical reagent or crosslinker, preferably a modified antigen-binding protein that binds to the crosslinker;
wherein the drug in step 2) is optionally modified or unmodified, preferably modified.

**11.** The method of claim 10, wherein said crosslinker preferably has a compound of the following formula ($L_2$):

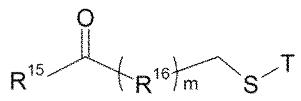

($L_2$)

T is selected from H, tert-butyl, acetyl, n-propionyl, isopropionyl, triphenylmethyl, methoxymethyl, 2-(trimethylsilyl) ethoxymethyl, preferably H or acetyl;
$R^{15}$ is selected from the group consisting of a hydrogen atom, a halogen, a hydroxyl group, a cyano group, an alkyl group, an alkoxy group and a cycloalkyl group;
$R^{16}$ is selected from the group consisting of alkyl, cycloalkyl and heterocyclic;
m is 0-5, preferably 1-3.

**12.** The method of claim 10, wherein said crosslinker represented by the formula (L2) is the compounds of the formula ($L_3$):

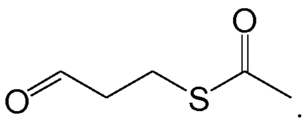

($L_3$)

**13.** The method of claim 10, wherein the crosslinker has a maleimide group or a haloacetyl moiety;
wherein the crosslinker having a maleimide group is preferably selected from the group consisting of SMCC, LC-SMCC, KMUA, GMBS, EMCS, MBS, AMAS, SMPH, SMPB, and PMPI, more preferably SMCC;
the crosslinker bearing a haloacetyl moiety is preferably selected from the group consisting of SIAB, SIA, SBA and SBAP, more preferably SIAB.

**14.** The method of any one of claims 1 to 13, wherein the drug is selected from the group consisting of a toxin, a chemotherapeutic agent, a growth inhibitor, a tubulin inhibitor, an antibiotic, a radioisotope, and a cytotoxic agent.

**15.** The method of any one of claims 1 to 14, wherein the drug is selected from the group consisting of a maytansinnoid derivative, an auristatin derivative, a camptothecin alkaloid; wherein the maytansinoid derivative is preferably selected from DM1, DM3, DM4; the auristatin derivative is preferably selected from MMAE, MMAF; the camptothecin alkaloid is preferably selected from CPT, 10-hydroxy-CPT, CPT-11, SN-38 and topotecan, more preferably SN-38.

**16.** The method of any one of claims 1 to 15, wherein the drug is selected from the group consisting of a compound represented by the following formula (Dr):

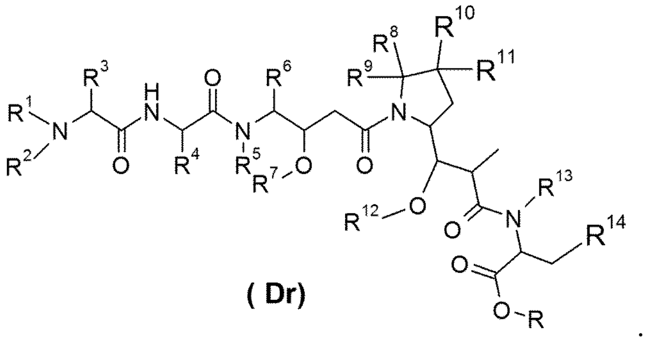

( Dr)

or a tautomer, a mesomer, a racemate, an enantiomer, a diastereomer, or a mixture thereof, or a pharmaceutically acceptable salt thereof, wherein:

R, $R^1$-$R^7$ are selected from the group consisting of a hydrogen atom, a halogen, a hydroxyl group, a cyano group, an alkyl group, an alkoxy group, and a cycloalkyl group;
at least one of $R^8$-$R^{11}$ is selected from the group consisting of halogen, alkenyl, alkyl and cycloalkyl, the remaining are hydrogen atoms;
or any two of $R^8$-$R^{11}$ form cycloalkyl groups, and the remaining two groups are selected from a hydrogen atom, an alkyl group and a cycloalkyl group;
$R^{14}$ is selected from aryl or heteroaryl, and the aryl or heteroaryl is optionally further substituted with a substituent selected from the group consisting of a hydrogen atom, a halogen, a hydroxyl group, an alkyl group, an alkoxy group, and a cycloalkyl group;
$R^{12}$-$R^{13}$ are selected from a hydrogen atom, an alkyl group or a halogen;
preferably, wherein the formula (Dr) is a compound of the following formula (I):

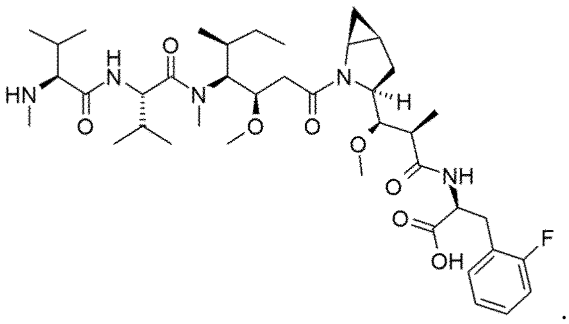

(I)

**17.** The method of any one of claims 1 to 16, wherein the drug is a modified compound, preferably is a compound of the following formula ($L_1$-Dr):

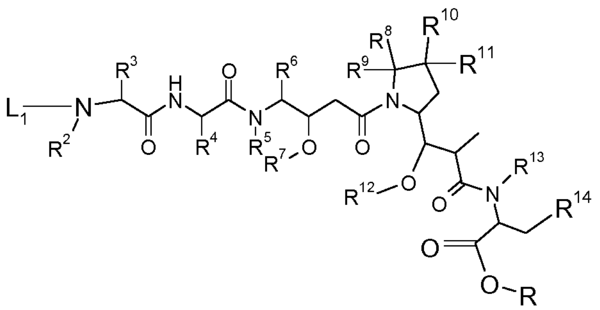

($L_1$-Dr)

wherein,
the $L_1$ structure is as follows:

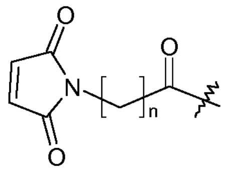

;

preferably MC;

n is 2-6, preferably 2-5;
R, $R^2$-$R^7$ are selected from the group consisting of a hydrogen atom, a halogen, a hydroxyl group, a cyano group, an alkyl group, an alkoxy group, and a cycloalkyl group;
at least one of $R^8$-$R^{11}$ is selected from the group consisting of halogen, alkenyl, alkyl and cycloalkyl, the remaining are hydrogen atoms;
or any two of $R^8$-$R^{11}$ form a cycloalkyl group, and the remaining two groups are selected from a hydrogen atom, an alkyl group and a cycloalkyl group;
$R^{12}$-$R^{13}$ are selected from a hydrogen atom, an alkyl group or a halogen;
$R^{14}$ is selected from aryl or heteroaryl, and the aryl or heteroaryl is optionally further substituted with a substituent selected from the group consisting of a hydrogen atom, a halogen, a hydroxyl group, an alkyl group, an alkoxy group, and a cycloalkyl group;
more preferably, wherein the formula ($L_1$-Dr) is a compound represented by (II):

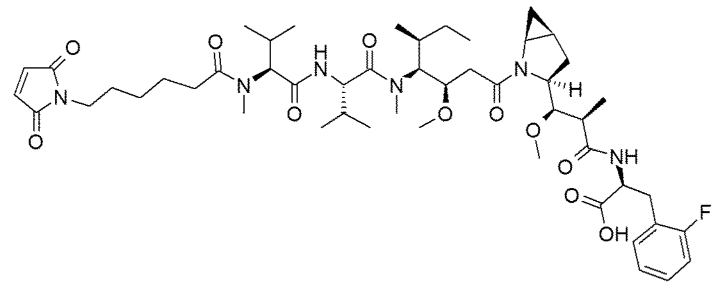

.

(II)

18. The method of claim 1, wherein the antigen-binding protein is selected from the group consisting of a humanized antibody, a murine antibody, a human antibody, a chimeric antibody, a single chain antibody, a bispecific antibody, preferably a humanized antibody.

19. The method of claim 1, wherein the antigen-binding protein is a monoclonal antibody or antigen binding fragment selected from the group consisting of Fab, F(ab')2, scFv fragments.

20. The method of any one of claims 1 to 19, wherein the antigen-binding protein binds to one or more polypeptides selected from the group consisting of HER2, HER3, CD33, VEGF, VEGFR, VEGFR-2, CD152, CD40, TNF, IL-1, IL-5, IL-17, IL-6R, IL-1, IL-2R, BLYS, OX40L, CTLA4, PCSK9, EGFR, c-Met, CD2, CD3, CD11a, CD19, CD30, CD38, CD20, CD52, CD60, CD80, CD86, TNF-$\alpha$, IL-12, IL-17, IL-23, IL-6, IL-1$\beta$, RSVF, IgE, RANK, BLyS, $\alpha 4\beta 7$, PD-1, CCR4, SLAMF7, GD2, CD21, CD79b, IL20R$\alpha$, shortenin, CD22, CD79a, CD72, IGF-1R and RANKL, or antigen-binding fragments thereof; preferably EGFR, c-Met, or an antigen-binding fragment thereof.

21. The method according to any one of claims 1 to 2, wherein the antigen-binding protein is selected from the group consisting of: Humira (adalimumab), Avastin (bevacizumab), Erbitux (cetuximab), Herceptin (Trastuzumab), Perjeta (Pertuzumab), Vectibix (Panibizumab), Theraloc (Netuzumab), Yervoy (Ipilimumab), Opdivo (Navolumab), Lucentis (Ranibizumab), Enbrel (Enacept), Myoscint (Imciromab pentetate), ProstaScint (Capromab pendetide), Remicade (Infliximab), ReoPro (Abciximab), Rituxan (rituximab), Simulect (Basiliximab), Synagis (Palivizumab), Verluma (Nofetumomab), Xolair (Omalizumab), Zenapax (Daclizumab), Cimzia (certolizumab), Zevalin (Ibritumomab), Orthoclone (Morommonab), Panorex (Edrecolomab), Mylotarg (Gemtuzumab), Soliris (Eculizumab), CNTO1275 (ustekinumab), Amevive (Alefacept), Raptiva (Efalizumab), Tysabri (Natalizumab), Acternra (Tocilizumab), Orencia (Abatacept), Arcalyst (Rilonacep), Stelara (Ustekinumab), Removab (Catumaxomab), Simponi (Golimumab), Ilaris (Canakinumab), Arzerra (Ofatumumab), Prolia (Denosumab), Benlysta (B elimumab), Nulojix (Belatacept), Eylea (Aflibercept), Campath (Alemtuzumab), CEA-Scan arcitumomab (fab fragment), Potelige (mogamulizumab), Abthrax (Raxibacumab), Gazyva (O binutuzumab), Lang Mu (Conbercept), Cyramza (Ramucirumab), Sylvant (Siltuximab), Entyvio (Vedolizumab), Keytruda (Pembrolizumab), Blincyto (Blinatumonab), Cosentyx (Secukinumab), Unituxin (Dinutuximab), Darzalex (Daratumumab), Praluent (Alirocumab), Repatha (Evolocumab), Portrazza (Necitumumab), Empliciti (Elotuzumab), Nucala (M epolizumab), Praxbind (Idarucizumab), Bexxar (Tositumomab and 1131 Tositumomab), or antigen-binding fragment thereof.

**22.** The method of any one of claims 1 to 21, wherein the antigen-binding protein is selected from the group consisting of an anti-EGFR antibody or antigen-binding fragment thereof, or an anti-c-Met antibody or antigen-binding fragment thereof;
wherein the anti-EGFR antibody or antigen-binding fragment thereof comprises LCDR1, LCDR2, LCDR3 region of SEQ ID NO:5, SEQ ID NO:6, SEQ ID NO:7, and variants thereof, HCDR1, HCDR2, HCDR3 region of SEQ ID NO:8, SEQ ID NO:9, SEQ ID NO:10 and variants thereof, preferably sequence of light chain of SEQ ID NO:1, and heavy chain of SEQ ID NO:2;
alternatively, wherein the anti-c-Met antibody or antigen-binding fragment thereof comprises LCDR1, LCDR2, LCDR3 region of SEQ ID NO:11 or SEQ ID NO:17, SEQ ID NO:12, SEQ ID NO:13, and variants thereof, preferably LCDR1 is SEQ ID NO:17, and HCDR1, HCDR2, HCDR3 region of SEQ ID NO:14, SEQ ID NO:15, SEQ ID NO:16 and variants thereof; the c-Met antibody or antigen-binding fragment thereof preferably comprises sequence of light chain of SEQ ID NO:3 and heavy chain of SEQ ID NO:4.

**23.** The method of any of claims 1-22, wherein the conductivity of the antigen-binding protein of step 1) is adjusted to less than 5 mS/cm prior to contacting with the ion exchange carrier.

**24.** The method of any one of claims 1 to 23, wherein the antigen-binding protein described in the step 1) is immobilized on an ion exchange carrier in a buffer having a pH of 5.5 to 7.0, preferably 6.3.

**25.** The method of claim 24, wherein said buffer is selected from the group consisting of phosphate buffer, acetate buffer, citrate buffer, succinate buffer, preferably phosphate buffer.

**26.** The method of any one of claims 1 to 25, wherein the immobilized antigen-binding protein of step 2) is coupled to a drug, and a coupling reaction is carried out by slowly flowing the drug through the ion exchange carrier to control the molar ratio of the drug to the antigen-binding protein in an amount of less than 6:1 and a flow rate of 0.2-2 ml/min.

**27.** The method of any one of claims 1 to 26, wherein the eluting of step 2) comprises stepwise elution using buffers with different salt concentrations, the pH of the buffer is 5.0-6.5, preferably 5.5.

**28.** The method of claim 27, wherein the buffer is selected from the group consisting of phosphate buffer, acetate buffer, citrate buffer, succinate buffer, preferably citrate buffer.

**29.** The method of any one of claims 1 to 28, wherein the stepwise elution comprises a first step elution and a second step elution, wherein the salt concentration of the first elution is 100-140 mM, preferably 110 mM, the salt concentration of the second elution is 150-200 mM, preferably 180 mM.

**30.** The method of any of claims 1 to 29, wherein said step 1) comprises:

a) binding the antigen-binding protein to the crosslinker to obtain a modified antigen-binding protein;
b) immobilizing the modified antigen-binding protein on an ion exchange carrier to form a immobilized antigen-binding protein;
c) adding a deprotecting agent, and the deprotecting agent is preferably $NH_2OH \cdot HCL$.

**31.** The method of claim 30, wherein the antigen-binding protein binds to the crosslinker at a temperature of 20 to 40 °C.

**32.** The method of claim 30, wherein the binding of the antigen-binding protein to the crosslinker is performed in a buffer

having a pH of 4.0 to 5.5, preferably at a pH of 4.3; the buffer is preferably an acetate buffer, more preferably acetate buffer containing acetonitrile.

**33.** The method of any of claims 1 to 29, wherein said step 1) comprises:

A) immobilizing the antigen-binding protein on an ion exchange carrier to form a immobilized antigen-binding protein;
B) adding a reducing agent to reduce disulfide bridges of the immobilized antigen-binding protein;

wherein the reducing agent is preferably selected from the group consisting of TCEP, DTT, mercaptoethylamine, Ac-Cys, more preferably TCEP.

**34.** The method of claim 33, wherein the reduction reaction of step B is performed at a temperature 25 to 45°C, preferably 40°C.

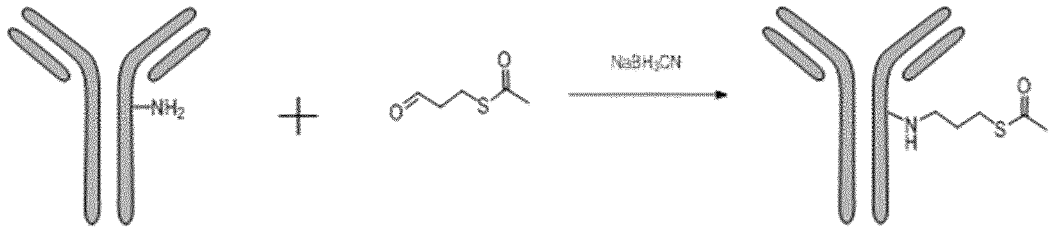


Monoclonal antibody Crosslinker Intermediate I

Figure 1

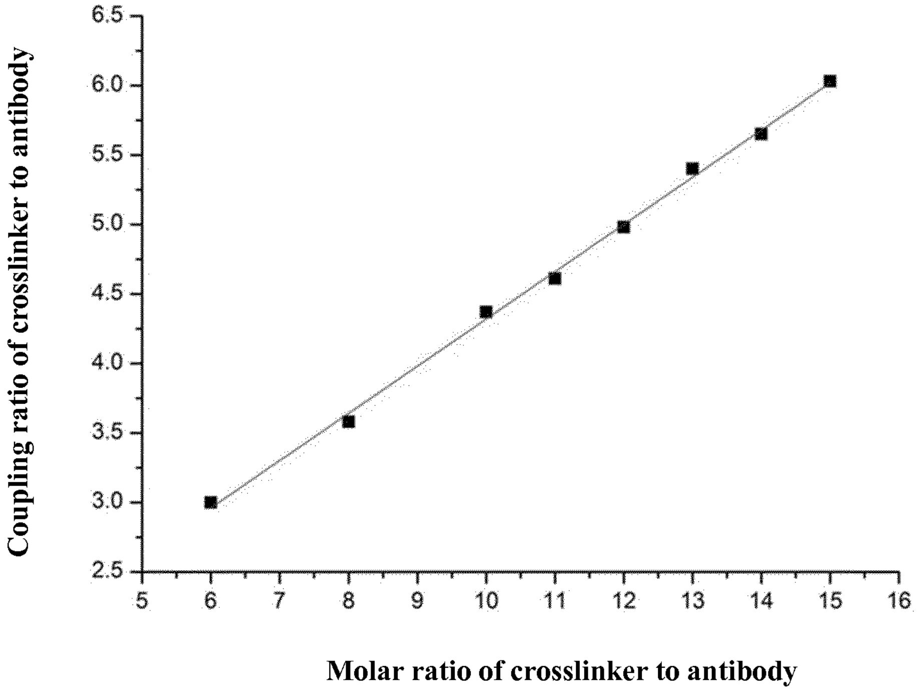


Figure 2

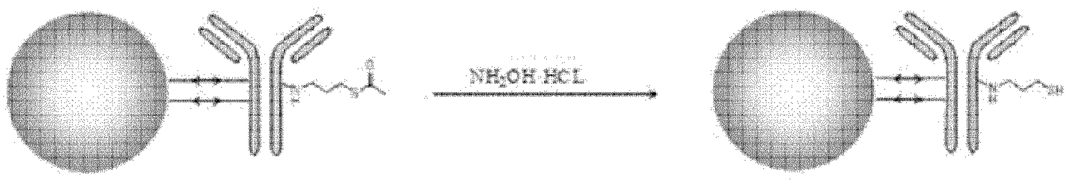


Intermediate I Intermediate II

Figure 3

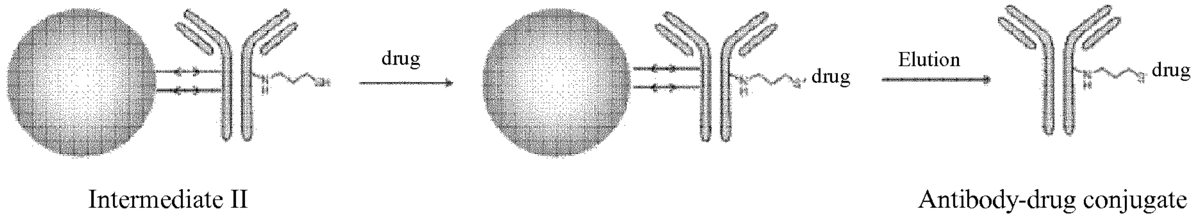


Figure 4

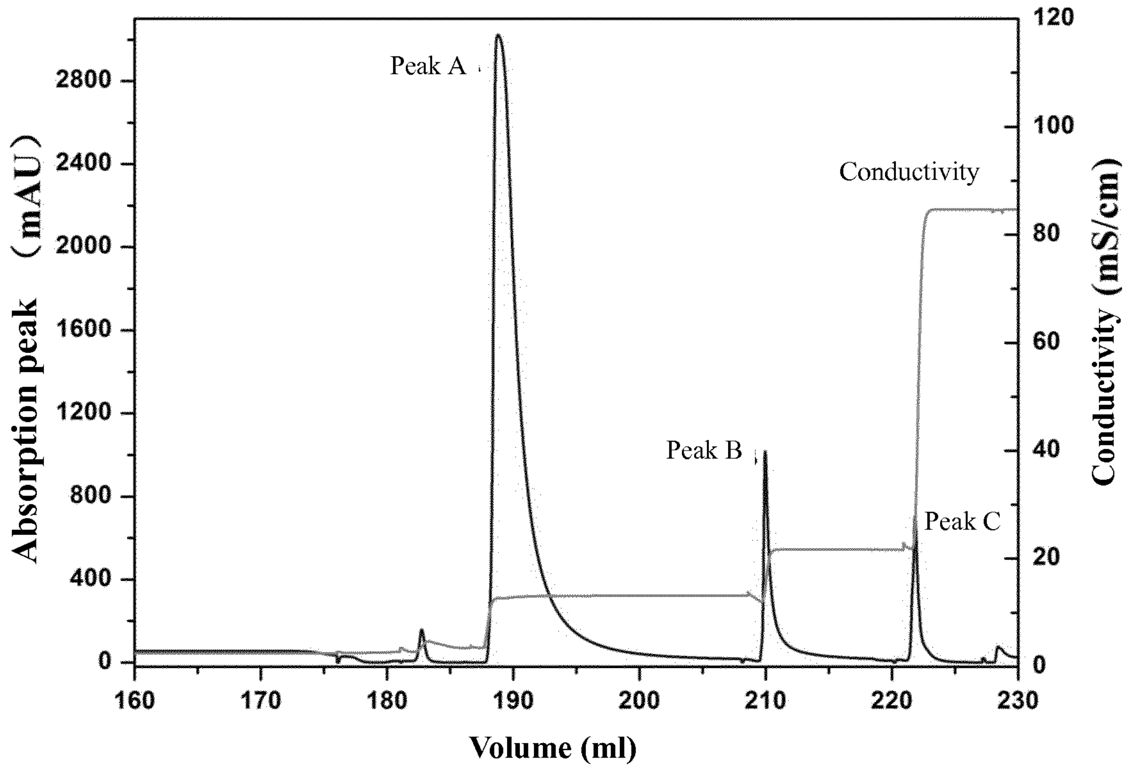


Figure 5

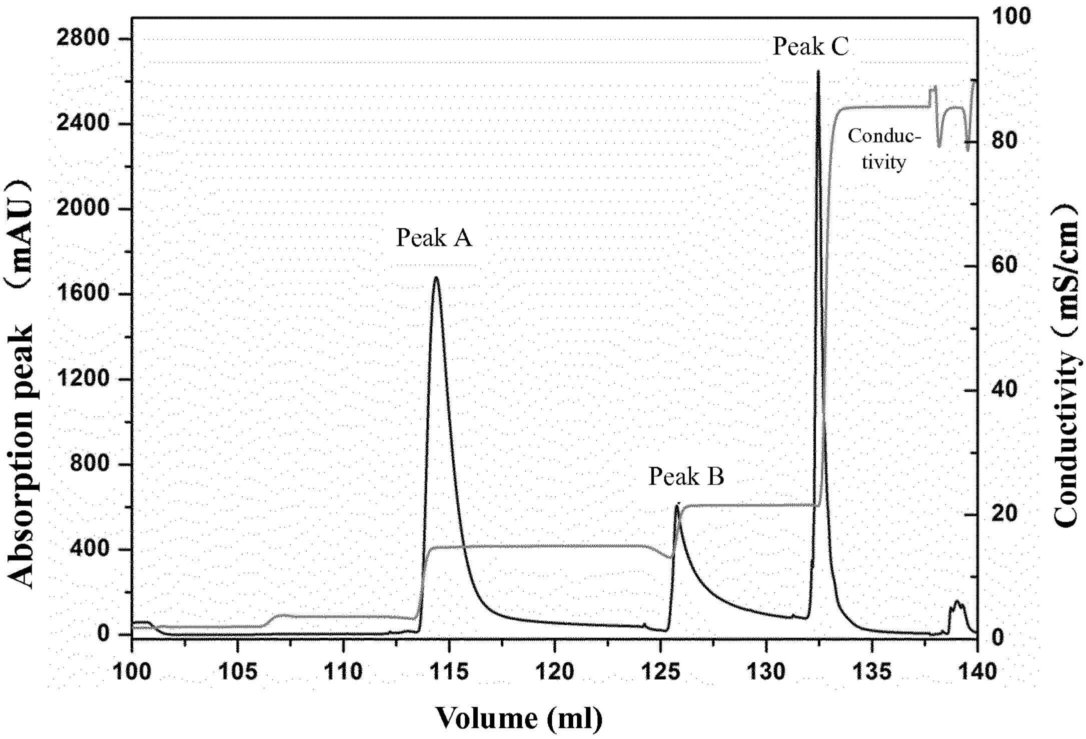
Peak A
Peak B
Peak C
Conduc-
tivity
Absorption peak （mAU）
Conductivity（mS/cm）
Volume (ml)
0
400
800
1200
1600
2000
2400
2800
0
20
40
60
80
100
100
105
110
115
120
125
130
135
140

Figure 6

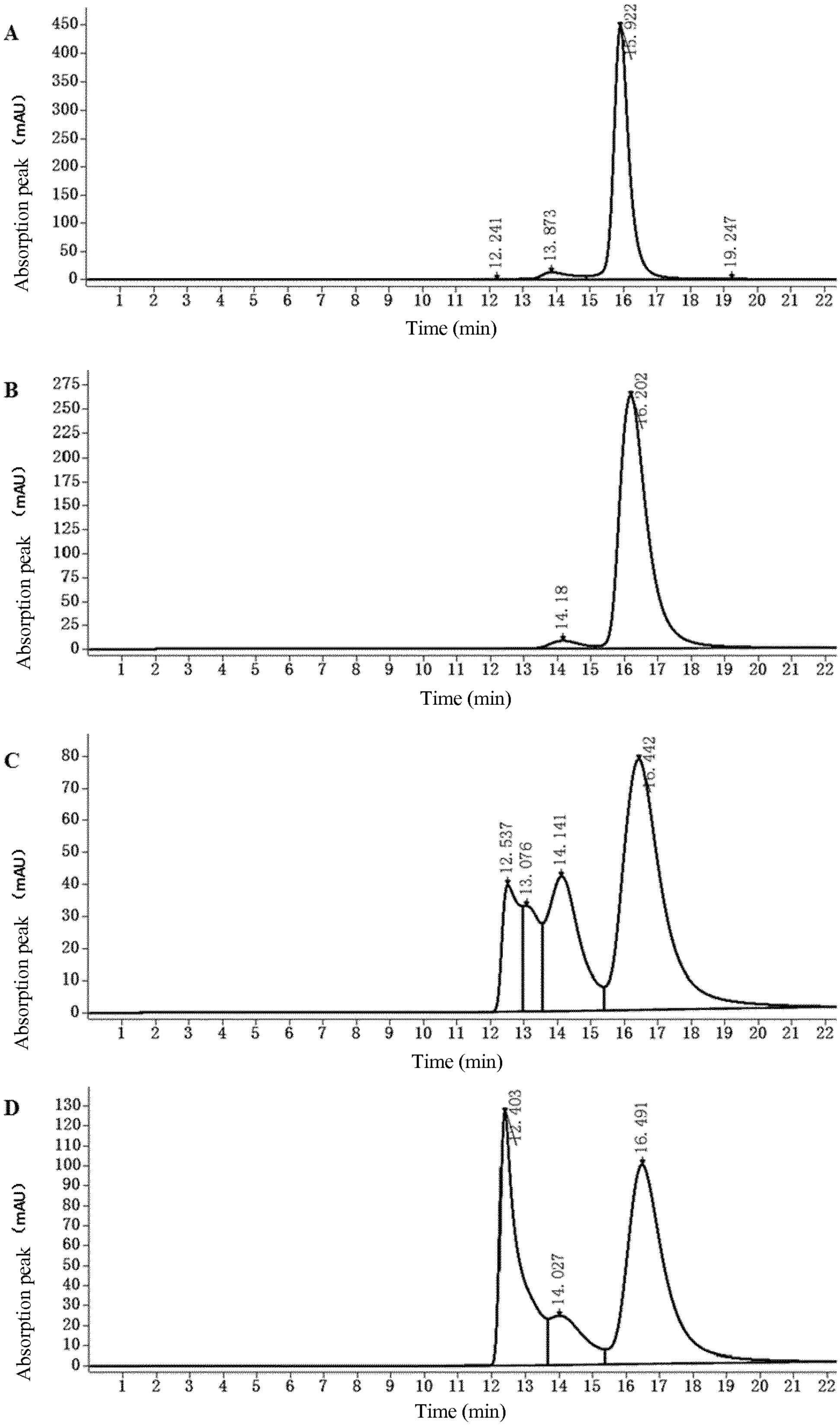
A
B
C
D
Absorption peak （mAU）
Time (min)
12. 241
13. 873
19. 247
14. 18
12. 537
13. 076
14. 141
14. 027
16. 491

Figure 7

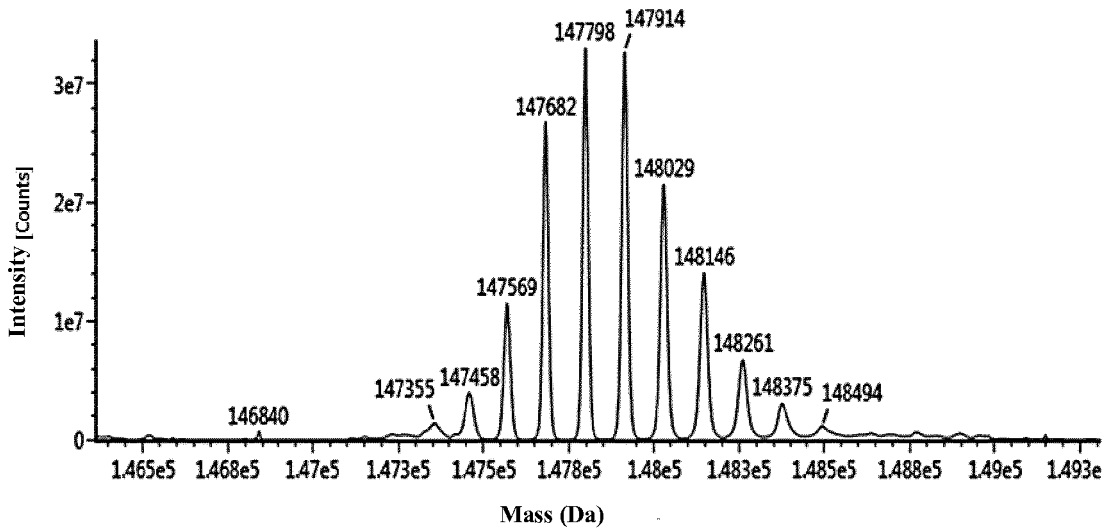


Figure 8

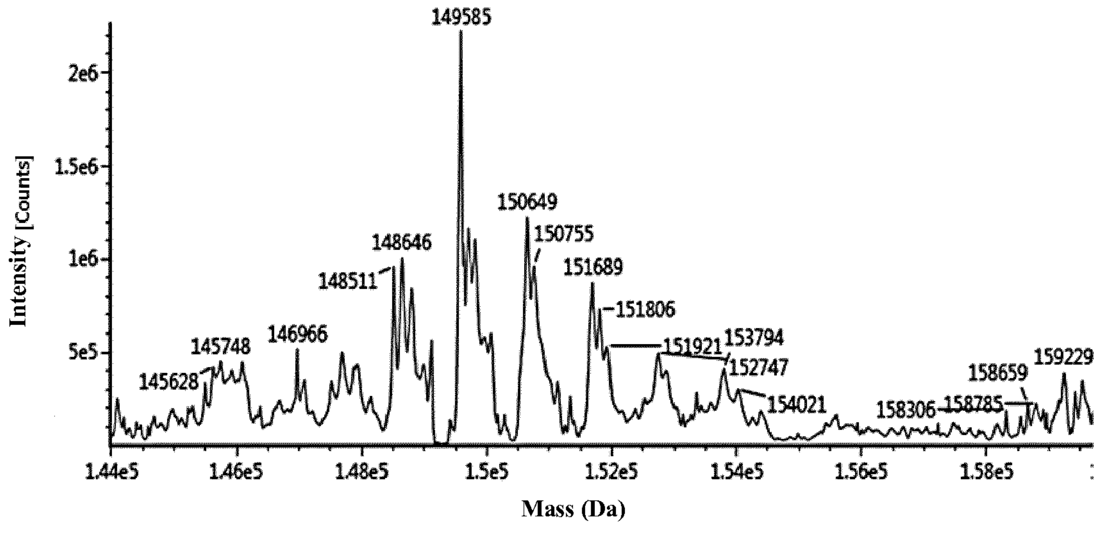


Figure 9

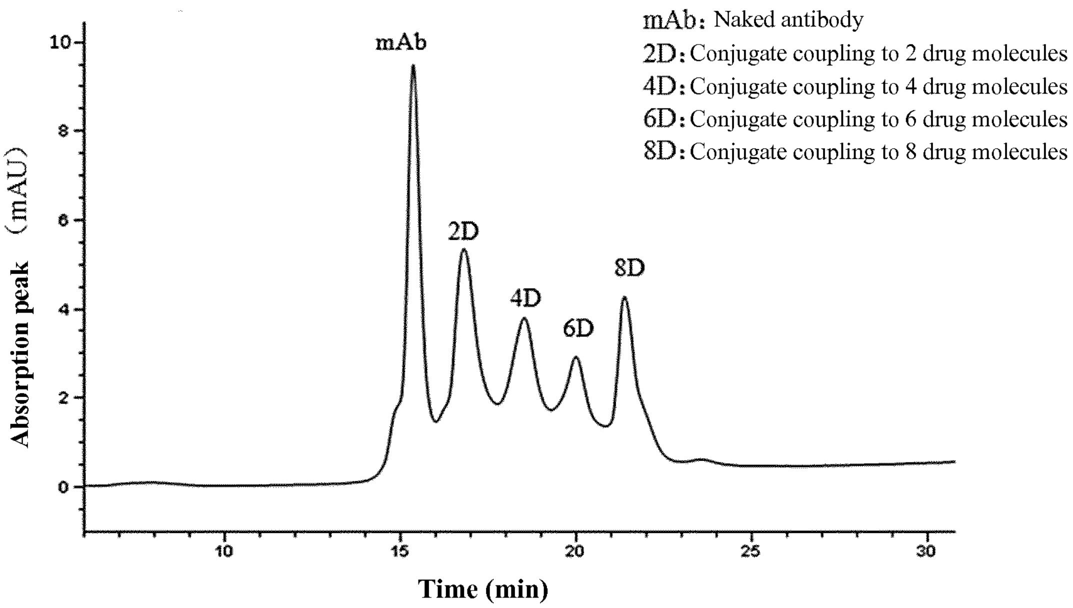


Figure 10

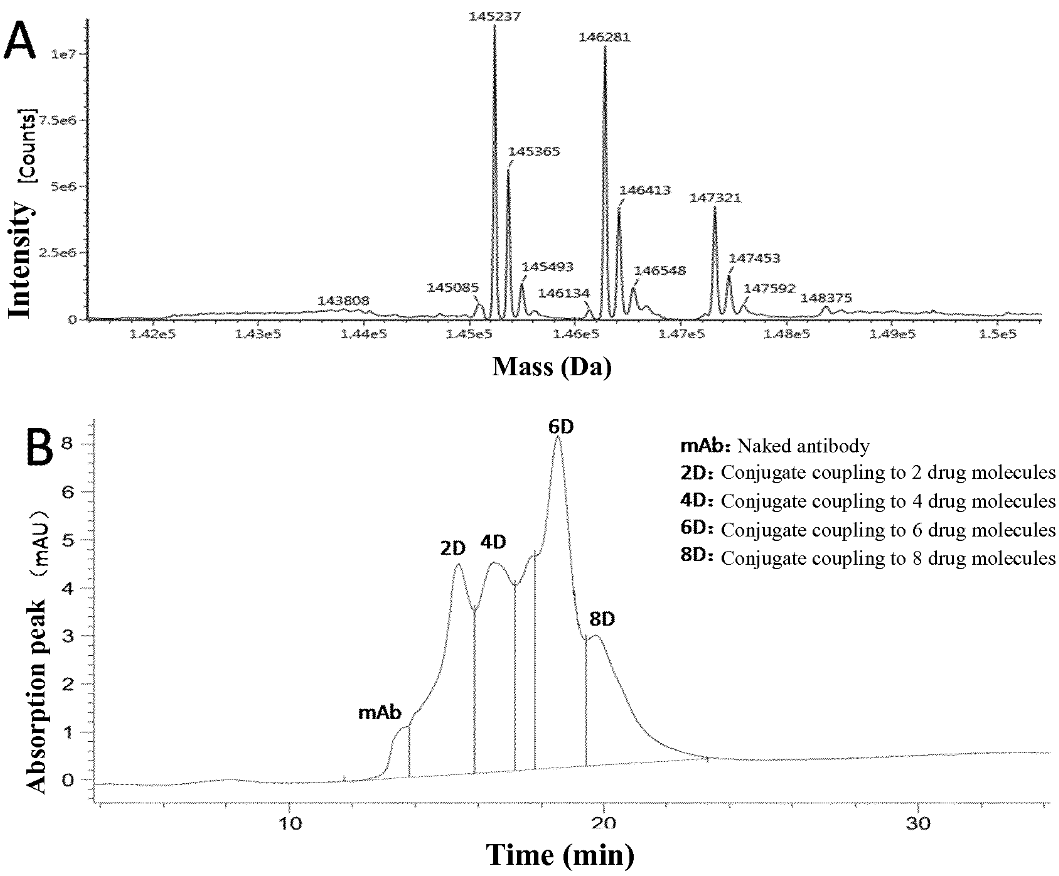


Figure 11

# INTERNATIONAL SEARCH REPORT

International application No.
PCT/CN2018/081080

**A. CLASSIFICATION OF SUBJECT MATTER**

A61K 39/00 (2006.01) i; C07K 16/18 (2006.01) i; C07K 1/04 (2006.01) i; C07K 1/18 (2006.01) i

According to International Patent Classification (IPC) or to both national classification and IPC

B. FIELDS SEARCHED

Minimum documentation searched (classification system followed by classification symbols)

A61K, C07K

Documentation searched other than minimum documentation to the extent that such documents are included in the fields searched

Electronic data base consulted during the international search (name of data base and, where practicable, search terms used)

CNABS, CPRSABS, DWPI, SIPOABS, CNTXT, WOTXT, USTXT, EPTXT, CNKI, WANFANG DATA, PubMed, ISI Web of Knowledge, BAIDU, DUXIU, NATIONAL BIO-SEQUENCE RETRIEVAL SYSTEM DATABASE OF CHINESE PATENT, GenBank, EBI-EMBL, 江苏恒瑞医药股份有限公司, 上海恒瑞医药有限公司, 刘宇鹏, 张小飞, 梁志, 石瑞君, 钟金, 刘洵, 陶维康, 张连山, 孙飘扬, 抗体药物偶联物, 离子交换, 固相, 固定, antibody drug conjugate, ADC, ion exchange, cation, anion, IEX, solid, fixed, DAR, sequence search

C. DOCUMENTS CONSIDERED TO BE RELEVANT

| Category* | Citation of document, with indication, where appropriate, of the relevant passages | Relevant to claim No. |
|---|---|---|
| Y | CN 104208719 A (BEIJING MABWORKS BIOTECH CO., LTD.), 17 December 2014 (17.12.2014), claims 1-10, description, paragraph 0020, and embodiments 2, 6 and 7 | 1-34 |
| Y | CN 105579066 A (ADC BIOTECHNOLOGY LTD.), 11 May 2016 (11.05.2016), claims 1-23 | 1-34 |
| Y | WO 2016067013 A1 (ADC BIOTECHNOLOGY LTD.), 06 May 2016 (06.05.2016), claims 1-25 | 1-34 |
| Y | CN 101267841 A (IMMUNOGEN INC.), 17 September 2008 (17.09.2008), claims 1-41 | 1-34 |
| Y | WO 2014055842 A1 (IMMUNOGEN, INC.), 10 April 2014 (10.04.2014), claims 8-76 | 1-34 |
| A | CN 106188293 A (JIANGSU HENGRUI MEDICINE CO., LTD.; SHANGHAI HENGRUI MEDICINE CO., LTD.), 07 December 2016 (07.12.2016), entire document | 1-34 |

☐ Further documents are listed in the continuation of Box C. ☒ See patent family annex.

* Special categories of cited documents:

"A" document defining the general state of the art which is not considered to be of particular relevance

"E" earlier application or patent but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| 30 May 2018 | 14 June 2018 |

| Name and mailing address of the ISA | Authorized officer |
|---|---|
| State Intellectual Property Office of the P. R. China<br>No. 6, Xitucheng Road, Jimenqiao<br>Haidian District, Beijing 100088, China<br>Facsimile No. (86-10) 62019451 | YANG, Jiaqian<br>Telephone No. 53961939 |

Form PCT/ISA/210 (second sheet) (January 2015)

# INTERNATIONAL SEARCH REPORT
Information on patent family members

International application No.
PCT/CN2018/081080

| Patent Documents referred in the Report | Publication Date | Patent Family | Publication Date |
|---|---|---|---|
| CN 104208719 A | 17 December 2014 | CN 104208719 B | 03 May 2017 |
| CN 105579066 A | 11 May 2016 | US 2016067352 A1 | 10 March 2016 |
| | | DK 2988785 T3 | 05 February 2018 |
| | | AU 2014259160 A1 | 19 November 2015 |
| | | EP 2988785 B1 | 01 November 2017 |
| | | ES 2658420 T3 | 09 March 2018 |
| | | GB 2513405 A | 29 October 2014 |
| | | MX 2015014935 A | 02 June 2016 |
| | | GB 201307574 D0 | 12 June 2013 |
| | | JP 2016519116 A | 30 June 2016 |
| | | CA 2910064 A1 | 30 October 2014 |
| | | WO 2014174316 A1 | 30 October 2014 |
| | | EP 2988785 B8 | 04 April 2018 |
| | | EP 2988785 A1 | 02 March 2016 |
| | | KR 20160003080 A | 08 January 2016 |
| WO 2016067013 A1 | 06 May 2016 | CN 107106702 A | 29 August 2017 |
| | | GB 201419185 D0 | 10 December 2014 |
| | | CA 2965891 A1 | 06 May 2016 |
| | | US 2017326251 A1 | 16 November 2017 |
| | | JP 2017537975 A | 21 December 2017 |
| | | EP 3223851 A1 | 04 October 2017 |
| CN 101267841 A | 17 September 2008 | US 2011021744 A1 | 27 January 2011 |
| | | KR 20080034476 A | 21 April 2008 |
| | | ES 2533992 T3 | 16 April 2015 |
| | | US 8933205 B2 | 13 January 2015 |
| | | ZA 200801564 B | 25 July 2012 |
| | | EC SP088212 A | 26 March 2008 |
| | | JP 5738248 B2 | 17 June 2015 |
| | | SI 1928503 T1 | 30 November 2012 |
| | | NZ 595430 A | 31 May 2013 |
| | | CA 2794554 A1 | 01 March 2007 |
| | | EP 1928503 B1 | 03 October 2012 |
| | | EA 200800657 A1 | 29 August 2008 |
| | | HK 1120407 A1 | 05 July 2013 |
| | | AU 2006283726 B2 | 28 June 2012 |
| | | DK 1928503 T3 | 15 October 2012 |
| | | CA 2794554 C | 22 September 2015 |
| | | IL 189461 A | 31 July 2013 |
| | | MX 2008002597 A | 14 March 2008 |
| | | HR P20120794 T1 | 30 November 2012 |
| | | NZ 609752 A | 29 August 2014 |
| | | IL 220816 D0 | 30 August 2012 |
| | | IL 189461 D0 | 07 August 2008 |
| | | HK 1165329 A1 | 21 August 2015 |
| | | JP 5350792 B2 | 27 November 2013 |
| | | PT 1928503 E | 19 October 2012 |
| | | JP 6028001 B2 | 16 November 2016 |
| | | CR 9742 A | 29 September 2008 |
| | | CR 20150350 A | 20 August 2015 |
| | | EP 2662096 A1 | 13 November 2013 |

Form PCT/ISA/210 (patent family annex) (January 2015)

## INTERNATIONAL SEARCH REPORT
Information on patent family members

International application No.
PCT/CN2018/081080

| Patent Documents referred in the Report | Publication Date | Patent Family | Publication Date |
|---|---|---|---|
| | | KR 101301011 B1 | 30 August 2013 |
| | | SI EP1928503 T1 | 30 November 2012 |
| | | EP 2399609 B1 | 18 March 2015 |
| | | AU 2006283726 A1 | 01 March 2007 |
| | | IL 226985 A | 31 October 2016 |
| | | CN 102989000 A | 27 March 2013 |
| | | IL 220816 A | 31 August 2014 |
| | | WO 2007024536 A3 | 05 July 2007 |
| | | US 7811572 B2 | 12 October 2010 |
| | | US 9789204 B2 | 17 October 2017 |
| | | BR PI0615049 A2 | 26 April 2011 |
| | | CA 2893252 A1 | 01 March 2007 |
| | | CN 102989000 B | 20 April 2016 |
| | | JP 2009506032 A | 12 February 2009 |
| | | EP 1928503 A2 | 11 June 2008 |
| | | WO 2007024536 A2 | 01 March 2007 |
| | | JP 2013014604 A | 24 January 2013 |
| | | AU 2006283726 C1 | 07 May 2015 |
| | | EA 013327 B1 | 30 April 2010 |
| | | ES 2390826 T3 | 16 November 2012 |
| WO 2014055842 A1 | 10 April 2014 | US 2015306242 A1 | 29 October 2015 |
| CN 106188293 A | 07 December 2016 | US 2018110875 A1 | 26 April 2018 |
| | | KR 20170138451 A | 15 December 2017 |
| | | CN 106687480 A | 17 May 2017 |
| | | AU 2016248357 A1 | 26 October 2017 |
| | | CA 2982777 A1 | 20 October 2016 |
| | | EP 3284751 A1 | 21 February 2018 |
| | | TW 201638108 A | 01 November 2016 |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 201710202043
- CN 201710233373
- CN 201710342257
- CN 104208719 A
- CN 105579066 A
- CN 101087611 A
- CN 106029083 A
- CN 106467575 A
- WO 2016127790 A1
- WO 2015113476 A
- CN 106188293 A
- CN 201610526367
- US 7750116 B


### Non-patent literature cited in the description


- **BECK A ; REICHERT JM.** Antibody-drug conjugates: Present and future. *MAbs,* 2014, vol. 6, 15-17
- **MCCOMBS J R ; OWEN S C.** Antibody-drug conjugates: design and selection of linker, payload and conjugation chemistry. *The AAPS journal,* 2015, vol. 17, 339-351
- Site-directed antibody-drug conjugates for cancer therapy. **PANOWSKI S et al.** MAbs. Taylor & Francis, 2014, vol. 6, 34-45
- **WANG L et al.** Structural characterization of the maytansinoid-monoclonal antibody immunoconjugate, huN901-DM1, by mass spectrometry. *Protein science,* 2005, vol. 14, 2436-2446
- **HAMBLETT KJ et al.** Effects of drug loading on the antitumor activity of a monoclonal antibody-drug conjugate. *Clin Cancer Res,* 2004, vol. 10, 7063-7070
- **YU et al.** The biosynthetic gene cluster of the maytansinoid antitumor agent ansamitocin from Actinosynnema pretiosum. *PNAS,* 2002, vol. 99, 7968-7973

SEQUENCE LISTING

<110> JIANGSU HENGRUI MEDICINE CO., LTD.
SHANGHAI HENGRUI PHARMACEUTICAL CO., LTD

<120> Method for preparing Antibody-drug conjugate

<130> P19412490EP

<150> CN201710202043.1
<151> 2017-03-30

<150> CN201710233373.7
<151> 2017-04-11

<150> CN201710342257.9
<151> 2017-05-16

<160> 17

<170> PatentIn version 3.5

<210> 1
<211> 219
<212> PRT
<213> Artificial Sequence

<220>
<223> amino acid sequence of mAb001 light chain

<400> 1

```
Asp Ile Gln Met Thr Gln Ser Pro Ser Ser Leu Ser Ala Ser Val Gly
1               5                   10                  15

Asp Arg Val Thr Ile Thr Cys Arg Ser Ser Gln Asn Ile Val His Ser
            20                  25                  30

Asn Gly Asn Thr Tyr Leu Asp Trp Tyr Gln Gln Thr Pro Gly Lys Ala
        35                  40                  45

Pro Lys Leu Leu Ile Tyr Lys Val Ser Asn Arg Phe Ser Gly Val Pro
    50                  55                  60

Ser Arg Phe Ser Gly Ser Gly Ser Gly Thr Asp Phe Thr Phe Thr Ile
65                  70                  75                  80

Ser Ser Leu Gln Pro Glu Asp Ile Ala Thr Tyr Tyr Cys Phe Gln Tyr
                85                  90                  95

Ser His Val Pro Trp Thr Phe Gly Gln Gly Thr Lys Leu Gln Ile Thr
            100                 105                 110

Arg Thr Val Ala Ala Pro Ser Val Phe Ile Phe Pro Pro Ser Asp Glu
        115                 120                 125
```

```
Gln Leu Lys Ser Gly Thr Ala Ser Val Val Cys Leu Leu Asn Asn Phe
    130                 135                 140

Tyr Pro Arg Glu Ala Lys Val Gln Trp Lys Val Asp Asn Ala Leu Gln
145                 150                 155                 160

Ser Gly Asn Ser Gln Glu Ser Val Thr Glu Gln Asp Ser Lys Asp Ser
                165                 170                 175

Thr Tyr Ser Leu Ser Ser Thr Leu Thr Leu Ser Lys Ala Asp Tyr Glu
            180                 185                 190

Lys His Lys Val Tyr Ala Cys Glu Val Thr His Gln Gly Leu Ser Ser
        195                 200                 205

Pro Val Thr Lys Ser Phe Asn Arg Gly Glu Cys
    210                 215


<210>  2
<211>  453
<212>  PRT
<213>  Artificial Sequence

<220>
<223>  amino acid sequence of mAb001 heavy chain

<400>  2

Gln Val Gln Leu Gln Gln Ser Gly Ala Glu Val Lys Lys Pro Gly Ser
1               5                   10                  15

Ser Val Lys Val Ser Cys Lys Ala Ser Gly Tyr Thr Phe Thr Asn Tyr
            20                  25                  30

Tyr Ile Tyr Trp Val Arg Gln Ala Pro Gly Gln Gly Leu Glu Trp Ile
        35                  40                  45

Gly Gly Ile Asn Pro Thr Ser Gly Gly Ser Asn Phe Asn Glu Lys Phe
    50                  55                  60

Lys Thr Arg Val Thr Ile Thr Ala Asp Glu Ser Ser Thr Thr Ala Tyr
65                  70                  75                  80

Met Glu Leu Ser Ser Leu Arg Ser Glu Asp Thr Ala Phe Tyr Phe Cys
                85                  90                  95

Thr Arg Gln Gly Leu Trp Phe Asp Ser Asp Gly Arg Gly Phe Asp Phe
            100                 105                 110
```

```
Trp Gly Gln Gly Thr Thr Val Thr Val Ser Ser Ala Ser Thr Lys Gly
        115                 120                 125

Pro Ser Val Phe Pro Leu Ala Pro Ser Ser Lys Ser Thr Ser Gly Gly
    130                 135                 140

Thr Ala Ala Leu Gly Cys Leu Val Lys Asp Tyr Phe Pro Glu Pro Val
145                 150                 155                 160

Thr Val Ser Trp Asn Ser Gly Ala Leu Thr Ser Gly Val His Thr Phe
                165                 170                 175

Pro Ala Val Leu Gln Ser Ser Gly Leu Tyr Ser Leu Ser Ser Val Val
            180                 185                 190

Thr Val Pro Ser Ser Ser Leu Gly Thr Gln Thr Tyr Ile Cys Asn Val
        195                 200                 205

Asn His Lys Pro Ser Asn Thr Lys Val Asp Lys Lys Val Glu Pro Lys
    210                 215                 220

Ser Cys Asp Lys Thr His Thr Cys Pro Pro Cys Pro Ala Pro Glu Leu
225                 230                 235                 240

Leu Gly Gly Pro Ser Val Phe Leu Phe Pro Pro Lys Pro Lys Asp Thr
                245                 250                 255

Leu Tyr Ile Thr Arg Glu Pro Glu Val Thr Cys Val Val Val Asp Val
            260                 265                 270

Ser His Glu Asp Pro Glu Val Lys Phe Asn Trp Tyr Val Asp Gly Val
        275                 280                 285

Glu Val His Asn Ala Lys Thr Lys Pro Arg Glu Glu Gln Tyr Asn Ser
    290                 295                 300

Thr Tyr Arg Val Val Ser Val Leu Thr Val Leu His Gln Asp Trp Leu
305                 310                 315                 320

Asn Gly Lys Glu Tyr Lys Cys Lys Val Ser Asn Lys Ala Leu Pro Ala
                325                 330                 335

Pro Ile Glu Lys Thr Ile Ser Lys Ala Lys Gly Gln Pro Arg Glu Pro
            340                 345                 350

Gln Val Tyr Thr Leu Pro Pro Ser Arg Asp Glu Leu Thr Lys Asn Gln
        355                 360                 365
```

```
Val Ser Leu Thr Cys Leu Val Lys Gly Phe Tyr Pro Ser Asp Ile Ala
    370                 375                 380

Val Glu Trp Glu Ser Asn Gly Gln Pro Glu Asn Asn Tyr Lys Thr Thr
385                 390                 395                 400

Pro Pro Val Leu Asp Ser Asp Gly Ser Phe Phe Leu Tyr Ser Lys Leu
                405                 410                 415

Thr Val Asp Lys Ser Arg Trp Gln Gln Gly Asn Val Phe Ser Cys Ser
            420                 425                 430

Val Met His Glu Ala Leu His Asn His Tyr Thr Gln Lys Ser Leu Ser
        435                 440                 445

Leu Ser Pro Gly Lys
    450


<210>  3
<211>  218
<212>  PRT
<213>  Artificial Sequence

<220>
<223>  amino acid sequence of mAb002 light chain

<400>  3

Asp Ile Val Leu Thr Gln Ser Pro Asp Ser Leu Ala Val Ser Leu Gly
1               5                   10                  15

Glu Arg Ala Thr Ile Asn Cys Arg Ala Asp Lys Ser Val Ser Thr Ser
            20                  25                  30

Thr Tyr Asn Tyr Leu His Trp Tyr Gln Gln Lys Pro Gly Gln Pro Pro
        35                  40                  45

Lys Leu Leu Ile Tyr Leu Ala Ser Asn Leu Ala Ser Gly Val Pro Asp
    50                  55                  60

Arg Phe Ser Gly Ser Gly Ser Gly Thr Asp Phe Thr Leu Thr Ile Ser
65                  70                  75                  80

Ser Leu Gln Ala Glu Asp Val Ala Val Tyr Tyr Cys Gln His Ser Arg
                85                  90                  95

Asp Leu Pro Pro Thr Phe Gly Gln Gly Thr Lys Leu Glu Ile Lys Arg
            100                 105                 110
```

Thr Val Ala Ala Pro Ser Val Phe Ile Phe Pro Pro Ser Asp Glu Gln
115 120 125

Leu Lys Ser Gly Thr Ala Ser Val Val Cys Leu Leu Asn Asn Phe Tyr
130 135 140

Pro Arg Glu Ala Lys Val Gln Trp Lys Val Asp Asn Ala Leu Gln Ser
145 150 155 160

Gly Asn Ser Gln Glu Ser Val Thr Glu Gln Asp Ser Lys Asp Ser Thr
165 170 175

Tyr Ser Leu Ser Ser Thr Leu Thr Leu Ser Lys Ala Asp Tyr Glu Lys
180 185 190

His Lys Val Tyr Ala Cys Glu Val Thr His Gln Gly Leu Ser Ser Pro
195 200 205

Val Thr Lys Ser Phe Asn Arg Gly Glu Cys
210 215

<210> 4
<211> 446
<212> PRT
<213> Artificial Sequence

<220>
<223> amino acid sequence of mAb002 light chain

<400> 4

Gln Val Gln Leu Val Glu Ser Gly Gly Gly Val Val Gln Pro Gly Arg
1 5 10 15

Ser Leu Arg Leu Ser Cys Ala Ala Ser Gly Phe Ser Leu Ser Asn Tyr
20 25 30

Gly Val His Trp Val Arg Gln Ala Pro Gly Lys Gly Leu Glu Trp Leu
35 40 45

Ala Val Ile Trp Ser Gly Gly Ser Thr Asn Tyr Ala Ala Ala Phe Val
50 55 60

Ser Arg Leu Thr Ile Ser Lys Asp Asn Ser Lys Asn Thr Val Tyr Leu
65 70 75 80

Gln Met Asn Ser Leu Arg Ala Glu Asp Thr Ala Val Tyr Tyr Cys Ala
85 90 95

Arg Asn His Asp Asn Pro Tyr Asn Tyr Ala Met Asp Tyr Trp Gly Gln

```
            100                 105                 110

Gly Thr Thr Val Thr Val Ser Ser Ala Ser Thr Lys Gly Pro Ser Val
        115                 120                 125

Phe Pro Leu Ala Pro Cys Ser Arg Ser Thr Ser Glu Ser Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Asn Phe Gly Thr Gln Thr Tyr Thr Cys Asn Val Asp His Lys
        195                 200                 205

Pro Ser Asn Thr Lys Val Asp Lys Thr Val Glu Arg Lys Cys Cys Val
    210                 215                 220

Glu Cys Pro Pro Cys Pro Ala Pro Pro Val Ala Gly Pro Ser Val Phe
225                 230                 235                 240

Leu Phe Pro Pro Lys Pro Lys Asp Thr Leu Met Ile Ser Arg Thr Pro
                245                 250                 255

Glu Val Thr Cys Val Val Val Asp Val Ser His Glu Asp Pro Glu Val
            260                 265                 270

Gln Phe Asn Trp Tyr Val Asp Gly Val Glu Val His Asn Ala Lys Thr
        275                 280                 285

Lys Pro Arg Glu Glu Gln Phe Asn Ser Thr Phe Arg Val Val Ser Val
    290                 295                 300

Leu Thr Val Val His Gln Asp Trp Leu Asn Gly Lys Glu Tyr Lys Cys
305                 310                 315                 320

Lys Val Ser Asn Lys Gly Leu Pro Ala Pro Ile Glu Lys Thr Ile Ser
                325                 330                 335

Lys Thr Lys Gly Gln Pro Arg Glu Pro Gln Val Tyr Thr Leu Pro Pro
            340                 345                 350
```

Ser Arg Glu Glu Met Thr Lys Asn Gln Val Ser Leu Thr Cys Leu Val
355 360 365

Lys Gly Phe Tyr Pro Ser Asp Ile Ala Val Glu Trp Glu Ser Asn Gly
370 375 380

Gln Pro Glu Asn Asn Tyr Lys Thr Thr Pro Pro Met Leu Asp Ser Asp
385 390 395 400

Gly Ser Phe Phe Leu Tyr Ser Lys Leu Thr Val Asp Lys Ser Arg Trp
405 410 415

Gln Gln Gly Asn Val Phe Ser Cys Ser Val Met His Glu Ala Leu His
420 425 430

Asn His Tyr Thr Gln Lys Ser Leu Ser Leu Ser Pro Gly Lys
435 440 445

<210> 5
<211> 16
<212> PRT
<213> Artificial Sequence

<220>
<223> sequence of mAb001-LCDR1

<400> 5

Arg Ser Ser Gln Asn Ile Val His Ser Asn Gly Asn Thr Tyr Leu Asp
1 5 10 15

<210> 6
<211> 7
<212> PRT
<213> Artificial Sequence

<220>
<223> sequence of mAb001-LCDR2

<400> 6

Lys Val Ser Asn Arg Phe Ser
1 5

<210> 7
<211> 9
<212> PRT
<213> Artificial Sequence

<220>
<223> sequence of mAb001-LCDR3

<400> 7

Phe Gln Tyr Ser His Val Pro Trp Thr

1 5

<210> 8
<211> 5
<212> PRT
<213> Artificial Sequence

<220>
<223> sequence of mAb001-HCDR1

<400> 8

Asn Tyr Tyr Ile Tyr
1 5

<210> 9
<211> 17
<212> PRT
<213> Artificial Sequence

<220>
<223> sequence of mAb001-HCDR2

<400> 9

Gly Ile Asn Pro Thr Ser Gly Gly Ser Asn Phe Asn Glu Lys Phe Lys
1 5 10 15

Thr

<210> 10
<211> 14
<212> PRT
<213> Artificial Sequence

<220>
<223> sequence of mAb001-HCDR3

<400> 10

Gln Gly Leu Trp Phe Asp Ser Asp Gly Arg Gly Phe Asp Phe
1 5 10

<210> 11
<211> 15
<212> PRT
<213> Mus musculus

<400> 11

Arg Ala Asn Lys Ser Val Ser Thr Ser Thr Tyr Asn Tyr Leu His
1 5 10 15

<210> 12
<211> 7
<212> PRT

<213>  Mus musculus

<400>  12

Leu Ala Ser Asn Leu Ala Ser
1               5

<210>  13
<211>  9
<212>  PRT
<213>  Mus musculus

<400>  13

Gln His Ser Arg Asp Leu Pro Pro Thr
1               5

<210>  14
<211>  5
<212>  PRT
<213>  Mus musculus

<400>  14

Asn Tyr Gly Val His
1               5

<210>  15
<211>  16
<212>  PRT
<213>  Mus musculus

<400>  15

Val Ile Trp Ser Gly Gly Ser Thr Asn Tyr Ala Ala Ala Phe Val Ser
1               5                   10                  15

<210>  16
<211>  12
<212>  PRT
<213>  Mus musculus

<400>  16

Asn His Asp Asn Pro Tyr Asn Tyr Ala Met Asp Tyr
1               5                   10

<210>  17
<211>  15
<212>  PRT
<213>  Artificial Sequence

<220>
<223>  optimized sequence of mAb002-LCDR1

<400>  17

Arg Ala Asp Lys Ser Val Ser Thr Ser Thr Tyr Asn Tyr Leu His